# EUROPÄISCHE PATENTSCHRIFT

(11) **EP 3 888 580 B1**
(45) Veröffentlichungstag und Bekanntmachung des Hinweises auf die Patenterteilung: **06.09.2023**
(21) Anmeldenummer: 21165983.4
(22) Anmeldetag: 30.03.2021
(51) Int. Cl.: A61B 18/12, A61B 18/00

(54) **ELEKTROCHIRURGIESYSTEM, ELEKTROCHIRURGISCHES INSTRUMENT UND ELEKTROCHIRURGIE-GENERATOR**
ELECTROSURGICAL SYSTEM, ELECTROSURGICAL INSTRUMENT AND ELECTROSURGICAL GENERATOR
SYSTÈME ÉLECTROCHIRURGICAL, INSTRUMENT ÉLECTROCHIRURGICAL ET GÉNÉRATEUR D'ÉLECTROCHIRURGIE

(30) Priorität: 30.03.2020 DE 102020108798; 31.03.2020 DE 102020108970; 30.10.2020 DE 102020128695
(43) Veröffentlichungstag der Anmeldung: 06.10.2021
(73) Patentinhaber: Olympus Winter & Ibe GmbH, 22045 Hamburg (DE)
(72) Erfinder: Janich, Fabian, 14469 Potsdam (DE); Kwik, Anne, 14165 Berlin (DE); Krüger, Jens, 15732 Eichwalde (DE); Lorenz, Karsten, 14513 Teltow (DE); Kadel, Michael, 12307 Berlin (DE); Breitsprecher, Frank, 12527 Berlin (DE)
(74) Vertreter: Eisenführ Speiser

(56) Entgegenhaltungen:
- US-A1- 2007 167 943
- US-A1- 2014 066 927
- US-A1- 2015 105 701

## Beschreibung

Die Erfindung betrifft ein Elektrochirurgiesystem mit einem Elektrochirurgie-Generator, der dazu konfiguriert ist, ein elektrochirurgisches Instrument mit hochfrequentem Wechselstrom zu versorgen. Die Erfindung betrifft außerdem ein Verfahren zum Betreiben eines Elektrochirurgiesystems sowie ein elektrochirurgisches Instrument und einen Elektrochirurgie-Generator.

Ein Elektrochirurgiesystem umfasst in der Regel einen Elektrochirurgie-Generator zum Erzeugen des hochfrequenten Wechselstroms. Der Elektrochirurgie-Generator hat in der Regel zwei oder mehr Ausgänge, an die ein elektrochirurgisches Instrument angeschlossen werden kann und zwischen denen im Betrieb eine hochfrequente Wechselspannung bereitgestellt wird. Dazu umfasst ein Elektrochirurgie-Generator in der Regel ein Hochspannungs-Netzteil, das im Betrieb einen Gleichstrom erzeugt, und einen Hochfrequenz-Teil, der mit dem Hochspannungs-Netzteil verbunden ist und im Betrieb aus dem Gleichstrom einen hochfrequenten Wechselstrom erzeugt.

An den Elektrochirurgie-Generator können typischerweise verschiedene elektrochirurgische Instrumente für unterschiedliche Aufgaben angeschlossen werden. Umgekehrt kann ein elektrochirurgisches Instrument unter Umständen auch mit verschiedenen Elektrochirurgie-Generatoren betrieben werden.

Mittels Elektrochirurgie kann biologisches Gewebe - also Körpergewebe - geschnitten, koaguliert (verödet) und/oder vaporisiert werden. Für die Elektrochirurgie werden typischerweise hochfrequente Wechselströme mit einer Frequenz zwischen 0,2 MHz und 3 MHz verwendet. Elektrochirurgische Instrumente sind typischerweise Handinstrumente, mit denen ein Arzt Körpergewebe koagulieren, ablatieren und/oder schneiden kann.

Elektrochirurgische Instrumente werden hierzu mit hochfrequenter elektrischer Energie gespeist, mit der Gewebe gezielt koaguliert oder geschnitten werden kann. Die hochfrequente elektrische Energie wird von einem Elektrochirurgie-Generator bereitgestellt und mittels des jeweils geeigneten elektrochirurgischen Instruments auf Körpergewebe angewandt. Je nach Anwendung sind spezielle Strom- und Spannungsverläufe notwendig, die dem Arzt in Form von Betriebsmodi (auch Modes genannt) zur Auswahl am Elektrochirurgie-Generator zur Verfügung gestellt werden. Diese Betriebsmodi können fest im Elektrochirurgie-Generator hinterlegt sein.

Aus WO 2011/032729 A1 ist eine einfach programmierbare Versorgungseinrichtung für ein elektrochirurgisches Instrument bekannt. Die einfache Programmierbarkeit resultiert aus definiert vorgegeben möglichen Zuständen eines Zustandsautomaten, die allerdings auch die Flexibilität der Versorgungseinrichtung einschränken.

In US 2014/0066927 A1 ist ein Elektrochirurgiesystem beschrieben, bei ein Instrument einen Speicher aufweist, welcher ein Script enthält, das in einen Script-Interpreter eines Generators des Elektrochirurgiesystems geladen werden kann. Das Script enthält Steuerbefehle und Parameter, die das Instrument für eine Verwendung mit dem Generator vorbereiten.

Der Erfindung liegt die Aufgabe zugrunde, ein Elektrochirurgiesystem zu schaffen, das verbesserte Möglichkeiten zur Abgabe von Informationen an einen Benutzer bietet.

Erfindungsgemäß werden hierzu ein Elektrochirurgiesystem, ein Elektrochirurgie-Generator, und ein elektrochirurgisches Instrument vorgeschlagen, die jeweils für sich und in Kombination miteinander einerseits flexibel kombinierbar sind und andererseits auf einen jeweiligen Anwendungsfall bzw. für ein jeweiliges elektrochirurgisches Instrument maßgeschneiderte Betriebsmodi ermöglichen. Außerdem werden ein Verfahren zum Betreiben des Elektrochirurgiesystems und des Elektrochirurgie-Generators vorgeschlagen.

Ein erfindungsgemäßes Elektrochirurgiesystem umfasst ein elektrochirurgisches Instrument und einen Elektrochirurgie-Generator, an den das elektrochirurgische Instrument im Betrieb angeschlossen ist.

### Zum Elektrochirurgie-Generator:

Der Elektrochirurgie-Generator verfügt über einen Prozessor und mindestens einen Generator-Datenspeicher, auf den der Prozessor zugreifen kann und in dem ein Betriebsprogramm zur Steuerung des Betriebs des Elektrochirurgie-Generators in Verbindung mit dem elektrochirurgischen Instrument gespeichert ist. Außerdem weist der Elektrochirurgie-Generator Ausgänge zum Anschließen eines elektrochirurgischen Instruments auf. Über die Ausgänge kann ein an den Elektrochirurgie-Generator im Betrieb angeschlossenes elektrochirurgisches Instrument mit elektrischer Energie, insbesondere mit hochfrequenter Wechselspannung oder hochfrequentem Wechselstrom versorgt werden. Der Elektrochirurgie-Generator weist außerdem ein Generator-Dateninterface für eine Datenkommunikation mit einem elektrochirurgischen Instrument, insbesondere einen Datenanschluss auf.

### Zum elektrochirurgischen Instrument:

Das elektrochirurgische Instrument weist einen Instrumenten-Datenspeicher auf, in dem wenigstens ein Datensatz gespeichert ist, der strukturierte Daten enthält, die als eine Vielzahl von parametrisierten Verweisen ausgeführt sind, die auf im Generator-Datenspeicher des Elektrochirurgie-Generators hinterlegte Steuerbefehle verweisen. Außerdem kann der Datensatz Werte für Betriebsparameter umfassen. Das elektrochirurgische Instrument weist außerdem ein Instrumenten-Dateninterface für eine Datenkommunikation mit dem Elektrochirurgie-Generator auf. Über das Instrumenten-Dateninterface kann der Elektrochirurgie-Generator im Betrieb strukturierte Daten aus dem im Instrumenten-Datenspeicher hinterlegten Datensatz auslesen. Darüber hinaus ermöglicht es das Instrumenten-Dateninterface, Datensätze mit strukturierten Daten, die als eine Vielzahl von parametrisierten Verweisen ausgeführt sind, in dem Instrumenten-Datenspeicher zu hinterlegen.

### Zum Betriebsprogramm:

Das Betriebsprogramm ist ausgebildet, neben Steuereingriffen an dem Elektrochirurgie-Generator auch Speicherzugriffe auf die strukturierten Daten in dem Instrumenten-Datenspeicher auszulösen und in dem Generator-Datenspeicher gespeicherte Steuerbefehle auszuführen, auf die die von den strukturierten Daten repräsentierten parametrisierten Verweise verweisen.

### Ablauf beim Betrieb des Elektrochirurgie-Generators:

Im Betrieb des Elektrochirurgie-Generators führt der Prozessor ein in dem Generator-Datenspeicher hinterlegtes Betriebsprogramm aus und steuert den Elektrochirurgie-Generator entsprechend dem Betriebsprogramm. Insbesondere löst der Prozessor durch das Betriebsprogramm gesteuert Steuereingriffe an dem Elektrochirurgie-Generator aus. Steuereingriffe umfassen dabei beispielsweise das Steuern der Abgabe von Energie an ein im Betrieb an den Elektrochirurgie-Generator angeschlossenes elektrochirurgisches Instrument. Steuereingriffe können aber auch die Abgabe von Signalen oder das Anzeigen von Informationen bewirken.

Die Steuereingriffe können durch das Betriebsprogramm unmittelbar gesteuert sein oder von den parametrisierten Verweisen abhängen, die in den strukturierten Daten enthalten sind, die in dem Instrumenten-Datenspeicher hinterlegt sind. Auch Werte für Parameter, die Steuereingriffe spezifizieren, können in den strukturierten Daten in dem Instrumenten-Datenspeicher enthalten sein.

Während des Betriebs des Elektrochirurgie-Generators werden die in den strukturierten Daten hinterlegten parametrisierten Verweise durch das Betriebsprogramm aufgerufen und diejenigen in dem Generator-Datenspeicher gespeicherten Steuerbefehle ausgeführt, auf die die von den strukturierten Daten repräsentierten parametrisierten Verweise verweisen. Falls die strukturierten Daten Werte für Betriebsparameter enthalten, werden die Steuerbefehle unter Berücksichtigung dieser in den strukturierten Daten angegebenen Betriebsparameterwerte ausgeführt.

Dazu greift der Elektrochirurgie-Generator während des Betriebs durch den Prozessor und das zugehörige Betriebsprogramm gesteuert auf die strukturierten Daten zu. Dies kann durch direkten Zugriff auf den Instrumenten-Datenspeicher erfolgen. Alternativ kann das Betriebsprogramm auch ausgebildet sein, eine Übertragung der strukturierten Daten aus dem Instrumenten-Datenspeicher in den Generator-Datenspeicher zu bewirken. Dies kann bei Inbetriebnahme des Elektrochirurgie-Generators oder beim Anschließen eines elektrochirurgischen Instruments erfolgen. Falls die strukturierten Daten bei Inbetriebnahme des Elektrochirurgie-Generators oder beim Anschließen eines elektrochirurgischen Instruments in den Generator-Datenspeicher ausgelesen werden, greift der Prozessor auf die dann in dem Generator-Datenspeicher vorliegenden strukturierten Daten zu.

### Zum Betriebsverfahren für das Elektrochirurgiesystem:

Erfindungsgemäß wird entsprechend auch ein Verfahren zum Betreiben eines Elektrochirurgiesystems mit einem elektrochirurgischen Instrument vorgeschlagen. Das Elektrochirurgiesystem weist einen Elektrochirurgie-Generator auf, an den das elektrochirurgische Instrument angeschlossen wird. Das elektrochirurgische weist einen Instrumenten-Datenspeicher auf. Der Elektrochirurgie-Generator verfügt über einen Prozessor und mindestens einen Generator-Datenspeicher. Der Prozessor führt zur Steuerung des Elektrochirurgie-Generators ein in dem Generator-Datenspeicher hinterlegtes Betriebsprogramm aus.

Erfindungsgemäß wird ein Datensatz erzeugt oder bereitgestellt, der strukturierte Daten enthält, wobei die strukturierten Daten als eine Vielzahl von parametrisierten Verweisen ausgeführt sind, die auf im Generator-Datenspeicher des Elektrochirurgie-Generators hinterlegte Betriebsvorgaben verweisen. Die Betriebsvorgaben sind insbesondere potentiell anzuwendende Steuerbefehle. Die strukturierten Daten können zusätzlich zu den parametrisierten Verweisen auch Betriebsparameterwerte umfassen. Betriebsparameterwerte können aber auch Teil der in dem Generator-Datenspeicher gespeicherten Betriebsvorgaben sein. In diesem Fall können die parametrisierten Verweise nicht nur auf Steuerbefehle, sondern auch auf Betriebsparameterwerte verweisen.

Der Datensatz mit strukturierten Daten wird oder ist in dem Instrumenten-Datenspeicher des elektrochirurgischen Instruments hinterlegt.

Bei Inbetriebnahme oder im Betrieb des Elektrochirurgie-Generators wird der im Instrumenten-Datenspeicher des elektrochirurgischen Instruments hinterlegte Datensatz mit strukturierten Daten von dem Elektrochirurgie-Generator ausgelesen und die in den strukturierten Daten hinterlegten parametrisierten Verweise werden durch das Betriebsprogramm gesteuert aufgerufen, um die im Elektrochirurgie-Generator hinterlegten Steuerbefehle unter Berücksichtigung von in den strukturierten Daten gegebenenfalls vorliegenden Betriebsparametern auszuführen, so dass der Elektrochirurgie-Generator in Abhängigkeit des Betriebsprogramms, der in dem Generator-Datenspeicher hinterlegten Betriebsvorgaben (z.B. Steuerbefehle) und der in den strukturierten Daten enthaltenen parametrisierten Verweise auf einzelne der in dem Generator-Datenspeicher hinterlegten Steuerbefehle gesteuert wird.

Betriebsprogramm und strukturierte Daten wirken beim Ausführen des Betriebsprogramms vorzugsweise derart zusammen, dass das Ausführen des Betriebsprogramms und das Aufrufen der strukturierten Daten durch das Betriebsprogramm den Betrieb des Elektrochirurgie-Generators in einem Betriebsmodus definiert.

Der Datensatz mit strukturierten Daten kann vorzugsweise manuell oder mit Hilfe einer Software erzeugt werden.

Vorzugsweise wird der im Instrumenten-Datenspeicher des elektrochirurgischen Instruments hinterlegte Datensatz mit strukturierten Daten nach dem Auslesen durch den Elektrochirurgie-Generator in dessen Generator-Datenspeicher abgelegt. Auf diese Weise braucht der Prozessor nur auf den Generator-Datenspeicher zuzugreifen, um sowohl das Betriebsprogramm, als auch die strukturierten Daten und die Betriebsvorgaben abzurufen, Gleichzeitig bleiben die Vorteile eines in dem Instrumenten-Datenspeicher abgelegten Datensatzes mit strukturierten Daten erhalten.

Das Betriebsverfahren kann auch Schritte aufweisen, in deren Rahmen auf dem elektrochirurgischen Instrument hinterlegte strukturierte Daten beim Auslesen durch den Elektrochirurgie-Generator oder nach dem Ablegen im Generator-Datenspeicher des Elektrochirurgie-Generators geändert werden. Dies kann beispielsweise das Hinzufügen von Zeilennummern und relativen Adressen beinhalten.

Die strukturierten Daten können Signalisierungsanweisungen umfassen, die im Betrieb des Elektrochirurgie-Generators in konfigurations- und/oder betriebsstatusabhängige akustische und/oder optische Benachrichtigungen an einen Benutzer umgesetzt werden.

Die strukturierten Daten können Definitionen für Aktivierungstöne umfassen.

Die strukturierten Daten können Bedingungen und Eigenschaften für eine bestimmte Benachrichtigung oder Fehlermeldung umfassen.

Vorzugsweise umfassen die strukturierten Daten Steuerzeichen für Textnachrichten repräsentierende Daten, wobei die Steuerzeichen die die Wiedergabe von Textnachrichten auf verschiedenen Anzeigen definieren und Textnachrichten im Betrieb des Elektrochirurgie-Generators in Abhängigkeit der Steuerzeichen und einer an dem Elektrochirurgie-Generator vorhandenen Anzeige für Text erzeugt werden.

### Zum Elektrochirurgiesystem:

Erfindungsgemäß wird auch ein Elektrochirurgiesystem vorgeschlagen, das ein elektrochirurgisches Instrument mit mindestens einem Instrumenten-Datenspeicher umfasst sowie einen Elektrochirurgie-Generator, an den das elektrochirurgische Instrument angeschlossen ist. Der Elektrochirurgie-Generator verfügt über einen Prozessor und mindestens einen Generator-Datenspeicher, auf den der Prozessor im Betrieb zugreifen kann. In dem Generator-Datenspeicher ist ein Betriebsprogramm hinterlegt, das der Prozessor zur Steuerung des Elektrochirurgie-Generators ausführen kann. Außerdem sind in dem Generator-Datenspeicher Betriebsvorgaben hinterlegt, die zumindest eine Vielzahl von Steuerbefehlen definieren. Diese Steuerbefehle definierenden Betriebsvorgaben werden im Rahmen dieser Beschreibung auch als Steuerbefehle bezeichnet.

In dem Instrumenten-Datenspeicher des elektrochirurgischen Instruments ist ein Datensatz mit strukturierten Daten hinterlegt. Die strukturierten Daten sind als eine Vielzahl von parametrisierten Verweisen ausgeführt, die auf die im Generator-Datenspeicher des Elektrochirurgie-Generators hinterlegten Steuerbefehle repräsentierenden Betriebsvorgaben verweisen. Die strukturierten Daten können außerdem Betriebsparameterwerte umfassen.

Der Elektrochirurgie-Generator ist ausgebildet, bei Inbetriebnahme oder während des Betriebs den im Instrumenten-Datenspeicher des elektrochirurgischen Instruments hinterlegten Datensatz mit strukturierten Daten auszulesen und während des Betriebs die in den strukturierten Daten hinterlegten parametrisierten Verweise durch das Betriebsprogramm aufzurufen, um diejenigen im Elektrochirurgie-Generator als Teil der Betriebsvorgaben hinterlegten Steuerbefehle auszuführen, auf die ein jeweiliger parametrisierter Verweis verweist. Dies kann unter Berücksichtigung von in den strukturierten Daten angegebenen Betriebsparameterwerten geschehen. Im Betrieb erfolgt die Steuerung des Elektrochirurgie-Generators somit in Abhängigkeit des Betriebsprogramms, der in dem Generator-Datenspeicher hinterlegten Betriebsvorgaben (insb. Steuerbefehle) und der in den strukturierten Daten enthaltenen parametrisierten Verweise auf einzelne der in dem Generator-Datenspeicher hinterlegten Betriebsvorgaben.

Der Elektrochirurgie-Generator kann über optische und/oder akustische Signalisierungs- und/oder Anzeigemittel verfügen, um einem Benutzer im Betrieb des Elektrochirurgie-Generators optische und/oder akustische Benachrichtigungen zukommen zu lassen. Falls der Elektrochirurgie-Generator über optische und/oder akustische Signalisierungs- und/oder Anzeigemittel verfügt, ist der Prozessor des Elektrochirurgie-Generators in Verbindung mit dem Betriebsprogramm dazu ausgebildet, über die Signalisierungs- und/oder Anzeigemittel auszugebenden Signale oder Benachrichtigungen anhand von in dem Instrumenten-Datenspeicher hinterlegten, Signalisierungsanweisungen repräsentierenden strukturierten Daten zu steuern. Die durch in dem Instrumenten-Datenspeicher hinterlegte strukturierte Daten repräsentierten Signalisierungsanweisungen definieren, wie und unter welchen Bedingungen welche Signale und/oder Information von dem Elektrochirurgie-Generator, an dem das elektrochirurgische Instrument betrieben wird, auszugeben sind.

Der Elektrochirurgie-Generator weist vorzugsweise einen Tongenerator und einen mit diesem zur Ausgabe akustischer Signale verbundenen Lautsprecher als Teil der Signalsisierungsmittel auf. Der Tongenerator ist mit dem Prozessor wirkverbunden und ausgebildet, durch den Prozessor in Abhängigkeit von Signalisierungsanweisungen repräsentierenden Daten in dem Instrumenten-Datenspeicher gesteuert, akustische Signale zu generieren und über den Lautsprecher auszugeben.

Vorzugsweise weist der Elektrochirurgie-Generator auch eine Anzeige als Signalisierungsmittel zum Anzeigen von Text und/oder Symbolen auf. Die Anzeige ist mit dem Prozessor verbunden und ausgebildet, durch den Prozessor in Abhängigkeit von Signalisierungsanweisungen repräsentierenden Daten in dem Instrumenten-Datenspeicher gesteuert, Textnachrichten auszugeben.

Der Prozessor ist - durch das Betriebsprogramm gesteuert - insbesondere dazu ausgebildet, die Signalisierungsanweisungen repräsentierenden Daten aus dem Instrumenten-Datenspeicher auszulesen und unter Berücksichtigung von Konfigurations- und/oder Statusinformationen derart zu verarbeiten, dass die Ausgabe von Signalen oder Informationen an einen Benutzer status- und/oder konfigurationsabhängig auf in dem elektrochirurgischen Instrument definierte Weise erfolgt. Konfigurationsinformationen können beispielsweise andere an einen Elektrochirurgie-Generator angeschlossene elektrochirurgische Instrumente betreffen. Statusinformationen können von Sensoren gewonnene Informationen über einen Betriebsstatus des Elektrochirurgie-Generators oder von dem Prozessor während des Betriebs erzeugte Statusinformationen sein.

Das Auslesen von Signalisierungsanweisungen aus dem Instrumenten-Datenspeicher kann beim Anschließen des elektrochirurgischen Instruments an den Elektrochirurgie-Generator erfolgen oder während des Betriebs des elektrochirurgischen Instruments.

Die status- und/oder konfigurationsabhängige Ausgabe von Signalen oder Informationen an einen Benutzer kann das Darstellen von Texten auf einer Anzeige oder das Ausgeben akustischer Signale beinhalten. Auf diese Weise ist es möglich, an einen Benutzer Warn- und/oder Statusinformationen, z.B. zum Betriebsmodus, zu Fehlermeldungen, über Betriebszustände etc. auszugeben.

Die Steuerbefehle und ggf. Betriebsparameterwerte, auf die die parametrisierten Verweise verweisen, sind Betriebsvorgaben, die in dem Generator-Datenspeicher gespeichert sind.

Der Generator-Datenspeicher enthält unabhängig vom Anschluss eines elektrochirurgischen Instruments eine theoretische beliebige Anzahl von Betriebsvorgaben, die von dem Betriebsprogramm aufgerufen werden können und den Betrieb des Elektrochirurgie-Generators beeinflussen können, für sich allein genommen, aber keine festen Betriebsabläufe vorgeben. Die Betriebsvorgaben können einerseits Steuerbefehle, Befehlsausdrücke und/oder bedingte Ausdrücke usw. sein, andererseits aber auch Betriebsparameterwerte wie zum Beispiel Werte für die Ausgangsspannung, den Strombetrag, Zeiteinstellungen, Impedanzwerte, Sollwerte usw. Insbesondere kann der Instrumenten-Datenspeicher auch Audiodateien enthalten, die akustische Signale definieren, die im Betrieb des Elektrochirurgie-Generators durch den Prozessor in Verbindung mit dem Betriebsprogramm und in Abhängigkeit der Signalisierungsanweisungen definierenden Daten in dem Datenspeicher des elektrochirurgischen Instruments gesteuert ausgegeben werden können.

Die Betriebsvorgaben in dem Instrumenten-Datenspeicher können als Bibliothek einer beliebigen Vielzahl von Betriebsvorgaben (wie z.B: Audiodateien für ggf. auszugebende akustische Signalen oder Ansteuerungsinformation für eine Textanzeige oder einen Frequenzgenerator) verstanden werden, auf die das Betriebsprogramm zugreifen kann. Allerdings erfolgt der Zugriff auf diese Betriebsvorgaben nicht unmittelbar, so dass die Betriebsweise des Elektrochirurgie-Generator allein durch die Betriebsvorgaben und das Betriebsprogramm noch nicht bestimmt ist.

Vielmehr greift der durch das Betriebsprogramm gesteuerte Prozessor beim Anschließen eines elektrochirurgischen Instruments und/oder während des Betriebs auf die Signalisierungsanweisungen repräsentierenden strukturierten Daten in dem Instrumenten-Datenspeicher zu, so dass die Ausgabe von Signalen oder Informationen an einen Benutzer auf in dem elektrochirurgischen Instrument definierte Weise erfolgt.

Vorzugsweise sind in dem Generator-Datenspeicher Audiodateien gespeichert, die der Tongenerator durch Prozessor in Abhängigkeit von Signalisierungsanweisungen repräsentierenden strukturierten Daten in dem Instrumenten-Datenspeicher gesteuert in akustische Signale umsetzen kann.

Vorzugsweise ist zusätzlich eine Datenstruktur vorgesehen, in der beliebige Datensätze mit strukturierten Daten gespeichert sind. Die strukturierten Daten enthalten Verweise auf potentiell anzuwendende Betriebsvorgaben wie Steuerbefehle oder Betriebsparameterwerte. Die durch die strukturierten Daten definierten parametrisierten Verweise werden im Betrieb des Elektrochirurgie-Generators von dem dann ablaufenden Betriebsprogramm aufgerufen und in konkret anzuwendende Betriebsvorgaben übersetzt, die das Betriebsprogramm dann anwenden kann. Die strukturierten Daten können insbesondere auch in dem Instrumenten-Datenspeicher gespeichert sein und Daten enthalten, die die Signalisierungsanweisungen repräsentieren.

Die konkrete Betriebsweise des Elektrochirurgie-Generators hängt somit vorzugsweise von drei verschiedenen Arten von gespeicherten Daten ab, nämlich
- von den das Betriebsprogramm definierenden Daten in dem Generator-Datenspeicher,
- von den potentiell anzuwendenden Betriebsvorgaben in dem Generator-Datenspeicher, die beispielsweise Steuerbefehle oder Betriebsparameterwerte definieren, und
- von den in der Datenstruktur gespeicherten Datensätzen mit strukturierten Daten, die während des Ablaufs des Betriebsprogramms durch dieses ausgelöst, den Aufruf der konkret anzuwendenden Betriebsvorgaben aus den in dem Instrumenten-Datenspeicher gespeicherten potentiell anzuwendenden Betriebsvorgaben bewirken .

Einige der in der Datenstruktur gespeicherten strukturierten Daten repräsentieren vorzugsweise Signalisierungsanweisungen, die im Betrieb des Elektrochirurgie-Generators von dem Prozessor des Elektrochirurgie-Generators in akustische oder optische Signale und/oder Benachrichtigungen für einen Benutzer umgesetzt werden. Die Umsetzung der Signalisierungsanweisungen in akustische und/oder optische Signale und/oder Benachrichtigungen für einen Benutzer kann status- und/oder konfigurationsabhängig auf in dem elektrochirurgischen Instrument definierte Weise erfolgen, da die Signalisierungsanweisungen repräsentierenden Daten in einer Datenstruktur auf dem elektrochirurgischen Instrument gespeichert sein können.

Die das Betriebsprogramm definierenden Daten und die die Betriebsvorgaben definierenden Daten in dem Instrumenten-Datenspeicher sowie die Datenstruktur mit den strukturierten Daten können unabhängig geändert und vorgegeben werden, mit der Einschränkung, dass die Datenformate kompatibel sind. Die Betriebsweise des Elektrochirurgie-Generators in einem jeweiligen Betriebsmodus kann somit durch Ändern des in dem Instrumenten-Datenspeicher gespeicherten Betriebsprogramms geändert werden, oder durch Ändern der in dem Instrumenten-Datenspeicher gespeicherten Betriebsvorgaben oder durch Ändern der strukturierten Daten in der Datenstruktur oder auch durch eine Kombination dieser Änderungen.

Das elektrochirurgische Instrument enthält den nichtflüchtigen Instrumenten-Datenspeicher (z.B. ein EEPROM oder ähnliches), aber keinen Prozessor. Auf dem Instrumenten-Datenspeicher befindet sich ein Datensatz mit strukturierten Daten. Die strukturierten Daten dieses Datensatzes sind zu der Datenstruktur in dem Generator-Datenspeicher des Elektrochirurgie-Generators kompatibel und können die Datenstruktur des Elektrochirurgie-Generators oder einen Teil der Datenstruktur des Elektrochirurgie-Generators bilden.

Die Datenstruktur kann somit in dem Generator-Datenspeicher vorliegen, der physischer Bestandteil des Elektrochirurgie-Generators ist oder sie wird durch den Inhalt des Instrumenten-Datenspeichers gebildet oder von einer Kombination aus Daten in dem Generator-Datenspeicher des Elektrochirurgie-Generators und in dem Instrumenten-Datenspeichers des elektrochirurgischen Instruments.

Der Prozessor des Elektrochirurgie-Generators ist mittels des Betriebsprogramms in dem Generator-Datenspeicher dazu konfiguriert, den Instrumenten-Datenspeicher des elektrochirurgischen Instruments auszulesen.

Der Prozessor des Elektrochirurgie-Generators kann mittels des Betriebsprogramms in dem Generator-Datenspeicher dazu konfiguriert sein, den Instrumenten-Datenspeicher des elektrochirurgischen Instruments auszulesen, nachdem das elektrochirurgische Instrument an den Elektrochirurgie-Generator angeschlossen wurde und bevor der Elektrochirurgie-Generator in einem Betriebsmodus betrieben wird. Der Prozessor des Elektrochirurgie-Generators kann mittels des Betriebsprogramms in dem Generator-Datenspeicher dazu konfiguriert sein, den Datensatz oder die Datensätze oder die in dem Datensatz oder den Datensätzen enthaltenen strukturierten Daten in eine Datenstruktur zu übertragen, die in dem Generator-Datenspeicher des Elektrochirurgie-Generators angelegt ist.

Der Prozessor des Elektrochirurgie-Generators kann mittels des Betriebsprogramms in dem Generator-Datenspeicher alternativ dazu konfiguriert sein, den Instrumenten-Datenspeicher während des Ablaufs des Betriebsprogramms - also während der Elektrochirurgie-Generator in einem Betriebsmodus betrieben wird - auszulesen. In dem Instrumenten-Datenspeicher ist somit kein Computerprogramm oder Algorithmus gespeichert, und die strukturierten Daten sind weder als Computerprogramm noch als ein mit einem Programm verbundener Algorithmus lesbar, sondern stellen vielmehr Verweise auf in dem Generator-Datenspeicher hinterlegte Betriebsvorgaben dar.

Die Struktur der strukturierten Daten erlaubt es, den parametrisierten Verweisen Zeilennummern zuzuordnen. Die Zeilennummern erlauben den gezielten Aufruf der diesen zugeordneten Verweise mittels des Betriebsprogramms. Die Verweise wiederum verweisen eindeutig auf konkrete Betriebsvorgaben in dem Generator-Datenspeicher oder alternativ auch auf andere Verweise in den strukturierten Daten.

Anstelle explizit im Datensatz gespeicherte Zeilennummern vorzusehen, kann auch vorgesehen sein, dass die Zeilennummern erst beim Auslesen eines Datensatzes erzeugt werden, und zwar anhand der Struktur der die Verweise repräsentierenden strukturierten Daten in einem Datensatz. Dies ist dann möglich, wenn die Verweise beispielsweise in einer vorgesehenen Reihenfolge in einem Datensatz gespeichert sind.

Die strukturierten Daten liegen vorzugsweise in einem speichereffizienten Binärformat vor, die beispielsweise in Form von Hexadezimalzahlen dargestellt werden können.

Vorzugsweise weist das Elektrochirurgiesystem eine Programmierschnittstelle oder mehrere Programmierschnittstellen auf, mittels der die Datenstruktur programmiert und auf ein elektrochirurgisches Instrument übertragen werden können. Es kann auch vorgesehen sein, dass das Betriebsprogramm und/oder die Betriebsvorgaben, insb. die Steuerbefehle, in dem Generator-Datenspeicher über eine Programmierschnittstelle geändert werden kann, beispielsweise auf dem Wege eines Softwareupdates des Elektrochirurgie-Generators via USB.

Bevorzugt ist ein Verfahren zum Betreiben eines Elektrochirurgie-Generators, bei dem Signalisierungsanweisungen repräsentierende Daten aus einem Instrumenten-Datenspeicher eines an den Elektrochirurgie-Generator angeschlossen elektrochirurgischen Instruments ausgelesen und im Betrieb des Elektrochirurgie-Generators in konfigurations- und/oder betriebsstatusabhängige akustische und/oder optische Benachrichtigungen an einen Benutzer umgesetzt werden.

Vorzugsweise enthalten Signalisierungsanweisungen für Textnachrichten repräsentierende Daten Steuerzeichen, die die Widergabe von Textnachrichten auf verschiedenen Anzeigen definieren, so das Textnachrichten im Betrieb des Elektrochirurgie-Generators in Abhängigkeit der Steuerzeichen und einer an dem Elektrochirurgie-Generator vorhandenen Anzeige für Text erzeugt werden.

Vorzugsweise werden die Signalisierungsanweisungen repräsentierenden Daten aus einem Instrumenten-Datenspeicher eines an den Elektrochirurgie-Generator angeschlossen elektrochirurgischen Instruments während des Betriebs des Elektrochirurgie-Generators ausgelesen. Alternativ können die Signalisierungsanweisungen repräsentierenden Daten auch beim Anschließen eines elektrochirurgischen Instruments an den Elektrochirurgie-Generator aus dem Instrumenten-Datenspeicher des elektrochirurgischen Instruments ausgelesen werden.

Vorzugsweise sind Betriebsvorgaben und eine Datenstruktur in voneinander unabhängigen Speicherbereichen des Generator-Datenspeichers auf dem Elektrochirurgie-Generator bereitgestellt, wobei die Datenstruktur Verweise auf die Betriebsvorgaben enthält. Der Prozessor des Elektrochirurgie-Generators greift vorzugsweise während des Ablaufs eines Betriebsprogramms indirekt auf einzelne Betriebsvorgaben zu, indem zunächst ein Zugriff auf parametrisierte Verweise in der Datenstruktur erfolgt und daraufhin ein Abruf derjenigen Betriebsvorgabe oder Betriebsvorgaben erfolgt, auf die ein jeweiliger Verweis verweist.

Vorzugsweise wird die Datenstruktur aus dem Instrumenten-Datenspeicher eines elektrochirurgischen Instruments ausgelesen, und zwar vorzugsweise nach Anschluss und vor einer Anwendung eines elektrochirurgischen Instruments. Die in der Datenstruktur auf dem elektrochirurgischen Instrument enthaltenen Datensätze mit strukturierten Daten können in eine Datenstruktur übertragen werden, die in dem Generator-Datenspeicher des Elektrochirurgie-Generators gespeichert ist.

Ein weiterer Aspekt der Erfindung ist ein Elektrochirurgie-Generator für ein Elektrochirurgiesystem der genannten Art. Der Elektrochirurgie-Generator besitzt Anschlüsse zum Anschließen eines elektrochirurgischen Instruments sowie einen Prozessor und mindestens einen Generator-Datenspeicher und optische und/oder akustische Signalisierungs- und/oder Anzeigemittel, um einem Benutzer im Betrieb des Elektrochirurgie-Generators optische und/oder akustische Benachrichtigungen zukommen zu lassen. Der Generator-Datenspeicher enthält ein Betriebsprogramm für den Prozessor zur Steuerung des Betriebs des Elektrochirurgie-Generators in Verbindung mit einem an diesem im Betrieb angeschlossenen elektrochirurgischen Instrument. Der Prozessor des Elektrochirurgie-Generators ist mittels des Betriebsprogramms dazu konfiguriert, im Betrieb des Elektrochirurgie-Generators die von den Signalisierungs- und/oder Anzeigemitteln auszugebenden Signale oder Benachrichtigungen abhängig von in einem Instrumenten-Datenspeicher eines angeschlossenen elektrochirurgischen Instruments hinterlegten, Signalisierungsanweisungen repräsentierenden strukturierten Daten zu steuern.

Der Elektrochirurgie-Generator weist vorzugsweise einen Tongenerator und einen mit diesem zur Ausgabe akustischer Signale verbundenen Lautsprecher als Signalisierungsmittel auf. Der Tongenerator ist mit dem Prozessor des Elektrochirurgie-Generators verbunden und ausgebildet, durch den Prozessordes Elektrochirurgie-Generators in Abhängigkeit von Signalisierungsanweisungen repräsentierenden Daten in dem Datenspeicher des elektrochirurgischen Instruments gesteuert akustische Signale zu generieren und über den Lautsprecher auszugeben.

Vorzugsweise weist der Elektrochirurgie-Generator auch eine Anzeige als Signalisierungsmittel zum Anzeigen von Text und/oder Symbolen auf. Die Anzeige ist mit dem Prozessor des Elektrochirurgie-Generators verbunden und ausgebildet, durch den Prozessor des Elektrochirurgie-Generators in Abhängigkeit von Signalisierungsanweisungen repräsentierenden Daten in dem Datenspeicher des elektrochirurgischen Instruments gesteuert, Textnachrichten auszugeben.

Ein derartiger Elektrochirurgie-Generator kann leicht mit verschiedenen unterschiedlichen elektrochirurgischen Instrumenten verwendet werden, wobei die Anpassung ein jeweiliges elektrochirurgisches Instrument allein durch entsprechende, Verweise repräsentierende Einträge in der Datenstruktur erfolgen kann, ohne dass die Betriebsvorgaben in dem Generator-Datenspeicher des Elektrochirurgie-Generators geändert werden müssen.

Ein weiterer Aspekt der Erfindung ist ein elektrochirurgisches Instrument, das einen Instrumenten-Datenspeicher aufweist, der Datensätze mit strukturierten Daten enthält, von denen wenigstens einige Signalisierungsanweisungen für durch einen Elektrochirurgie-Generator im Betrieb ausgebende akustische und/oder optische Signale repräsentieren.

Die strukturierten Daten können allgemein die Verweise auf in dem Generator-Datenspeicher gespeicherte Betriebsvorgaben enthalten. Ein derartiges elektrochirurgisches Instrument kann somit die nötigen Informationen enthalten, die eine auf das jeweilige elektrochirurgische Instrument abgestimmten Betriebsmodus erlauben, ohne dass dieser vollständig durch das elektrochirurgische Instrument definiert zu sein braucht.

Ein derartiges Elektrochirurgiesystem, ein derartiger Elektrochirurgie-Generator und ein derartiges elektrochirurgisches Instrument erlauben es, jeweils für sich und in Kombination miteinander, dass Benachrichtigungs- und Fehlerzustände sowie das Verhalten dieser Benachrichtigungen nicht schon zum Zeitpunkt der Entwicklung des Elektrochirurgie-Generators bekannt sein müssen. Auch ist eine nachträgliche Anpassung der Benachrichtigungs- und Fehlerzustände sowie das Verhalten dieser Benachrichtigungen leicht über ein Softwareupdate des elektrochirurgischen Instruments möglich. Sogar eine "live"-Optimierung der Benachrichtigungs- und Fehlerzustände sowie das Verhalten dieser Benachrichtigungen während Usability-Tests ist möglich.

Die Erfindung soll nun anhand von Ausführungsbeispielen mit Bezug auf die Figuren näher erläutert werden. Von den Figuren zeigt:
- Figur 1:: ein Elektrochirurgiesystem mit einem Elektrochirurgie-Generator und einem an diesem angeschlossenen elektrochirurgischen Instrument;
- Figur 2:: ein elektrochirurgisches Instrument;
- Figur 3:: ein schematisches Schaltbild eines Elektrochirurgie-Generators;
- Figur 4:: ein schematisches Bild eines Prozessors in Verbindung mit einem Generator-Datenspeicher des Elektrochirurgie-Generators aus Figur 3;
- Figur 5:: ein schematisches Bild einer alternativen Konfiguration eines Prozessors in Verbindung mit einem Generator-Datenspeicher des ElektrochirurgieGenerators aus Figur 3.

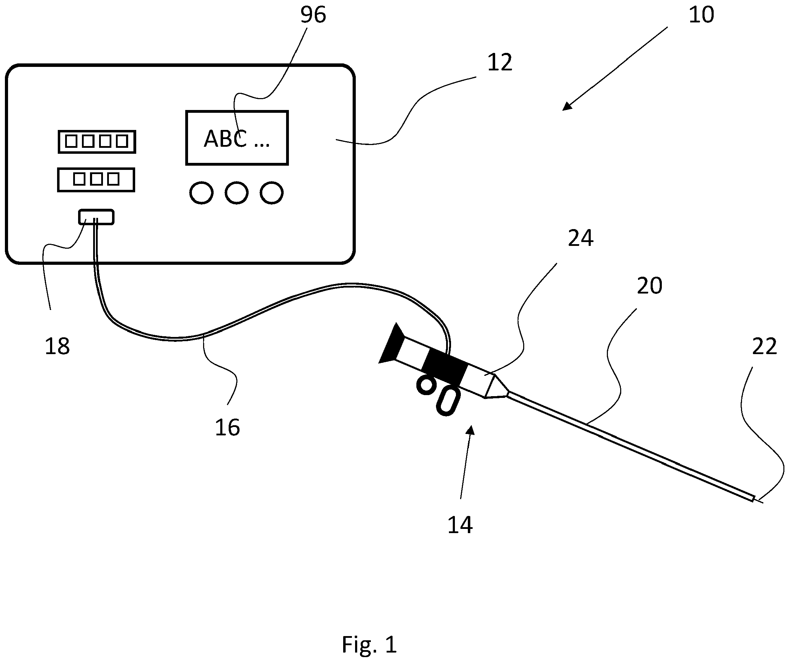

In Figur 1 ist ein Elektrochirurgiesystem 10 abgebildet. Das Elektrochirurgiesystem 10 umfasst einen Elektrochirurgie-Generator 12 und ein elektrochirurgisches Instrument 14. Das elektrochirurgische Instrument 14 ist über ein Anschlusskabel 16 mit elektrischen Aus- und Eingängen 18 des Elektrochirurgie-Generators 12 verbunden.

Das elektrochirurgische Instrument 14 weist einen Schaft 20 auf, an dessen Ende sich eine aktive Elektrode 22 befindet. Der Schaft 20 ist an einem Griff 24 des elektrochirurgischen Instruments 14 befestigt.

Das elektrochirurgische Instrument 14 weist einen Instrumenten-Datenspeicher 26 auf, der Datensätze 80 mit strukturierten Daten enthält, die parametrisierte Verweise auf Betriebsvorgaben repräsentieren, die in einem Generator-Datenspeicher 74 des Elektrochirurgie-Generators 12 gespeichert sind. Die strukturierten Daten repräsentieren außerdem Signalisierungsanweisungen, welche definieren, wie und unter welchen Bedingungen welche Signale und/oder Information von dem Elektrochirurgie-Generator 12, an dem das elektrochirurgische Instrument 14 betrieben wird, auszugeben sind. Der Instrumenten-Datenspeicher 26 ist nichtflüchtig und kann beispielsweise ein ROM (Read Only Memory) sein, beispielsweise ein EPROM (*electrically programmable read-only memory*)*,* insbesondere ein EEPROM (electrically erasable programmable read-only memory). Ein EEPROM ist ein nichtflüchtiger Datenspeicher, der gelöscht und neu beschrieben werden kann. Der Instrumenten-Datenspeicher 26 ist beispielsweise in dem Handgriff 24 des elektrochirurgischen Instruments 14 untergebracht.

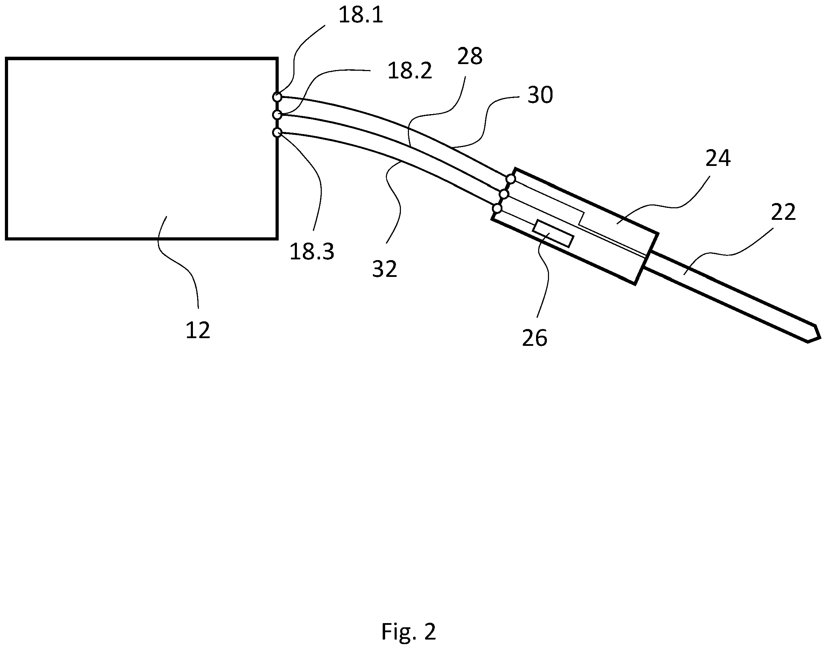

In dem Anschlusskabel 16 befinden sich sowohl Versorgungsleitungen 28 und 30 als auch wenigstens eine Datenleitung 32. Die Versorgungsleitungen 28 verbinden die aktive Elektrode 22 und eine nicht weiter dargestellte neutrale Elektrode mit den elektrischen Ausgängen 18.1 und 18.2 des Elektrochirurgie-Generators 12. Über die Datenleitung 32 ist der Instrumenten-Datenspeicher 26 mit einem entsprechenden Daten-Anschluss 18.3 des Elektrochirurgie-Generators 12 verbunden. Dies ist schematisch in Figur 2 dargestellt.

Die Datenleitung 32 in dem Anschlusskabel 16 sowie auch der Daten-Anschluss 18.3 können mehradrig bzw. mehrpolig sein. Zusätzlich zu der Datenleitung 32 oder anstelle der Datenleitung 32 kann auch eine drahtlose Schnittstelle für die Datenübertragung von dem elektrochirurgischen Instrument 14 zu dem Elektrochirurgie-Generator 12 vorgesehen sein. Eine solche drahtlose Schnittstelle kann beispielsweise eine Bluetooth Schnittstelle, eine NFC Schnittstelle oder eine RFID Schnittstelle sein.

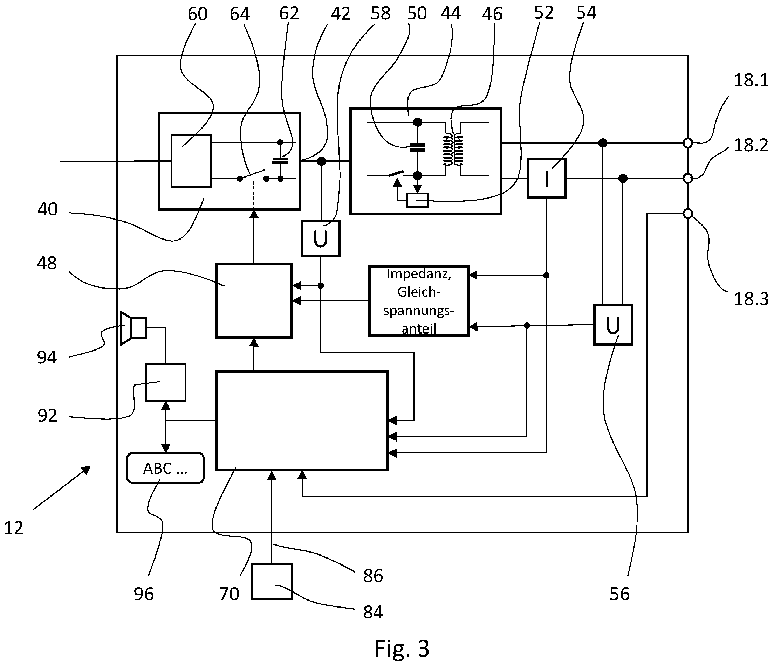

Die zum Betreiben des elektrochirurgischen Instruments 14 an dessen aktive Elektrode 22 und eine Gegenelektrode abzugebende Ausgangswechselspannung wird im Betrieb von dem Elektrochirurgie-Generator 12 bereitgestellt. Wie Figur 3 zu entnehmen ist, weist der Elektrochirurgie-Generator 12 hierzu ein Hochspannungsnetzteil 40 auf, das beispielsweise an das übliche öffentliche Stromversorgungsnetz angeschlossen werden kann und an seinem Ausgang 42 einen Hochspannungsgleichstrom mit einer Ausgangsgleichspannung bereitstellt. Dieser Ausgangsgleichstrom wird einem Hochfrequenzteil 44 des Elektrochirurgie-Generators 12 zugeführt. Der Hochfrequenzteil 44 des Elektrochirurgie-Generators 12 wirkt als Wechselrichter und erzeugt eine hochfrequente Ausgangswechselspannung, die über einen Ausgangstransformator 46 des Hochfrequenzteils 44 an die Ausgänge 18.1 und 18.2 des Elektrochirurgie-Generators 12 abgegeben wird. An die Ausgänge 18.1 und 18.2 des Elektrochirurgie-Generators 12 kann das elektrochirurgische Instrument 14 angeschlossen werden, wie es in den Figuren 1 und 2 dargestellt ist.

Um die Ausgangswechselspannung des Elektrochirurgie-Generators 12 zu steuern, ist eine Generatorsteuereinheit 48 vorgesehen, die die Ausgangswechselspannung an den Ausgängen 18.1 und 18.2 des Elektrochirurgie-Generators 12 auf Basis eines Ausgangswechselspannungsmaximalwertes derart steuert, dass beispielsweise ein eingestellter Ausgangsspannungsmaximalwert im Betrieb nicht überschritten wird.

Die Ausgangswechselspannung des Elektrochirurgie-Generators 12 - und damit auch der Ausgangswechselstrom und die Ausgangsleistung - können durch die von dem Hochspannungsnetzteil 40 erzeugte Ausgangsgleichspannung gesteuert werden.

Hierzu dient die Generatorsteuereinheit 48, die von dem Hochspannungsnetzteil 40 erzeugte Ausgangsgleichspannung so steuert, dass sich beispielsweise eine Ausgangswechselspannung oder ein Ausgangswechselstrom so ergibt, wie es ein jeweiliger Betriebsmodus des Elektrochirurgie-Generators zu einem jeweiligen Zeitpunkt verlangt.

Jeder Betriebsmodus definiert Maximalwerte für den Effektivwert der Ausgangswechselspannung über die Ausgänge 18.1 und 18.2, die Ausgangsspitzenspannung über die Ausgänge 18.1 und 18.2, den Ausgangswechselstrom an den Ausgängen 18.1 oder 18.2, den Gleichspannungsanteil an der Ausgangswechselspannung über die Ausgänge 18.1 und 18.2 sowie die Ausgangsgleichspannung des Hochspannungsnetzteils 40. Die durch einen jeweiligen Betriebsmodus definierten Maximalwerte können dabei situationsabhängig sein und sich während einer Anwendung ändern.

Die Generatorsteuereinheit 48 steuert das Hochspannungsnetzteil 40 in Abhängigkeit von durch einen jeweiligen Betriebsmodus definierten Maximalwerten und von im Betrieb von Erfassungseinheiten 54, 56 und 58 erfassten aktuellen Werten der Ausgangswechselspannung, der Ausgangsspitzenspannung, des Ausgangswechselstroms, des Gleichspannungsanteil an der Ausgangswechselspannung oder der Ausgangsgleichspannung oder einer Kombination von Werten dieser Parameter.

Die konkreten Maximalwerte und der zeitliche Ablauf für das Erzeugen der Ausgangswechselspannung des Elektrochirurgie-Generators 12 und deren Abhängigkeit von erfassten Momentanwerten hängen vom jeweiligen Betriebsmodus (Mode) ab, in dem der Elektrochirurgie-Generator 12 gerade betrieben wird.

Ein Betriebsmodus wird beispielsweise durch Betätigen eines entsprechenden Schalters, beispielsweise einem Fußschalter 84 durch einen Nutzer aufgerufen.

In einem jeweiligen Betriebsmodus wird der Betrieb des Elektrochirurgie-Generators 12 von einem Prozessor 70 in Verbindung mit einem in dem Generator-Datenspeicher gespeicherten Betriebsprogramm gesteuert. Der Prozessor 70 kann beispielsweise die Maximalwerte für die verschiedenen Betriebsparameter, wie die Ausgangswechselspannung, den Ausgangswechselstrom, die Ausgangsleistung oder aber auch den Gleichspannungsanteil an der Ausgangswechselspannung vorgeben, deren jeweils aktuelle Werte während des Betriebs des Elektrochirurgie-Generators 12 erfasst werden.

Der Prozessor 70 ist mit dem Generator-Datenspeicher 74 verbunden, in dem das Betriebsprogramm für den Elektrochirurgie-Generator 12 hinterlegt ist.

Der Prozessor 70 ist einerseits mit der Generatorsteuereinheit 48 verbunden, um die Ausgabe der im Betrieb an das elektrochirurgische Instrument abzugebenden Ausgangswechselspannung zu steuern. Andererseits ist der Prozessor 70 aber auch mit Signalisierungs- und Anzeigemitteln, nämlich im konkreten Fall mit einem Tongenerator 92 nebst Lautsprecher 94 und mit einer Anzeige 96 zum Anzeigen von Text und/oder Symbolen verbunden. Der Tongenerator 92 kann dazu ausgebildet sein, mittels eines Frequenzgenerators zu generieren oder in Form von Audiodateien vorgegebene akustische Signale über den Lautsprecher 94 wiederzugeben. Der Tongenerator kann auch zum Umsetzten von in Audiodateien gespeicherten Informationen in entsprechende Audiosignale ausgebildet sein.

Während des Ablaufs des in dem Generator-Datenspeicher 72 hinterlegten Betriebsprogramms 74 greift der Prozessor 70 an in dem Betriebsprogramm 74 hinterlegten Stellen auf für einen jeweiligen Betriebsmodus in dem Generator-Datenspeicher 74 ebenfalls hinterlegte Betriebsvorgaben 76 wie beispielsweise Daten, die Werte für Betriebsparameter repräsentieren und/oder Steuerbefehle zurück. Die in dem Generator Datenspeicher 72 hinterlegten Betriebsvorgaben 76 geben beispielsweise bestimmte Werte für die Ausgangsgleichspannung des Hochspannungsnetzteils oder die Ausgangswechselspannung, den Ausgangswechselstrom des Hochfrequenzteils oder Ähnliches vor. Die in dem Generator-Datenspeicher 72 hinterlegten Betriebsvorgaben 76 schließen aber auch bestimmte Steuerbefehle ein, wie beispielsweise *"if"* oder *"while"* oder *"true"* oder *"false".* Auf diese Weise können mit den in dem Generator-Datenspeicher 72 hinterlegten, Daten und Steuerbefehle repräsentierenden Betriebsvorgaben 76 beispielsweise solche Steueranweisungen definiert werden wie *"vergleiche den aktuellen Wert der Ausgangswechselspannung mit dem Betrag 200 und gib ein "true" aus, wenn der aktuelle Wert der Spannung kleiner oder gleich 200 ist und ein "false", wenn der aktuelle Wert größer ist als 200".* Andere Daten und Steuerbefehle können beispielsweise zu der Steueranweisung kombiniert werden, die besagt, dass die maximale Ausgangsgleichspannung des Hochspannungsnetzteils 100 Volt sein soll.

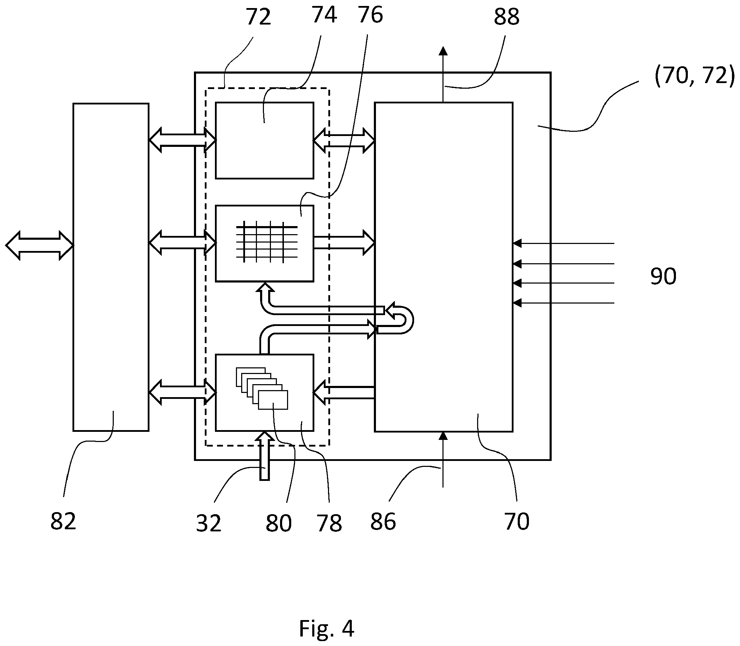

Um derartige Steueranweisungen zu generieren und auszuführen, greift der Prozessor 70 jedoch nicht direkt auf die Betriebsvorgaben 76 in dem Generator-Datenspeicher 72 zu, sondern ruft an entsprechenden Stellen des Betriebsprogramms 74 eine Datenstruktur 78 auf, in der für einen jeweiligen Betriebsmodus Verweise hinterlegt sind, die auf entsprechende Betriebsvorgaben 76 in dem Generator-Datenspeicher 72 verweisen, siehe z.B. Figur 4. Die Verweise können beispielsweise Zahlen sein, insbesondere 1-Byte große Binärzahlen, die auch als Hexadezimalzahlen dargestellt werden können, da diese wenig Speicherplatz erfordern. In diesem Falle können die Betriebsvorgaben 76 nach Art einer Tabelle strukturiert sein, in der jedem Verweis (also beispielsweise jeder Hexadezimalzahl) die entsprechenden Steuerbefehle oder Daten zugeordnet sind.

Um verschiedene Betriebsmodi zu verwirklichen, sind in der Datenstruktur 78 eine Vielzahl von Datensätzen 80 hinterlegt, von denen jeder ein oder mehrere Verweise enthält, denen aufgrund der Struktur des jeweiligen Datensatzes - insbesondere der Reihenfolge, in der die Verweise gespeichert sind - Zeilennummern zugeordnet werden können. Die einer Zeilennummer zugeordneten Verweise verweisen auf die entsprechenden Betriebsvorgaben 76 in dem Generator-Datenspeicher 72 und veranlassen den Prozessor 70, die entsprechenden Betriebsvorgaben 76 aus dem Generator-Datenspeicher 72 auszulesen, nachdem der Prozessor 70 zuvor auf die in der Datenstruktur 78 hinterlegte und die zugehörige Zeilennummer bezeichnete Einsprungadresse zugegriffen hat. Die in dem Generator-Datenspeicher 72 gespeicherten Betriebsvorgaben können beispielsweise Steueranweisungen, Steuerbefehle oder Parameterwerte sein, welche das Betriebsprogramm an der jeweiligen Stelle des Betriebsprogramms anwenden soll, an der sich in dem Betriebsprogramm 74 ein Verweis auf eine Zeilennummer in der Datenstruktur 78 befindet.

In der Datenstruktur 78 sind Verweise auf in dem Generator-Datenspeicher 72 gespeicherte Betriebsvorgaben 76 in einer geordneten Sequenz abgespeichert. Die Struktur eines jeweiligen Datensatzes in der Datenstruktur 78 erlaubt es, den Verweisen Zeilennummern nach Art von Adressen innerhalb der Datenstruktur 78 zuzuweisen, damit beispielsweise auch Sprünge oder Rücksprünge auf Verweise in der Datenstruktur 78 möglich sind und nicht lediglich ein streng sequentielles Abarbeiten der Verweise durch das Betriebsprogramm. Zeilennummern können als Einsprungadressen innerhalb der strukturierten Daten in der Datenstruktur 78 dienen, auf die der Prozessor 70 durch das Betriebsprogramm gesteuert zugreift. Die den Zeilennummern zugeordneten Verweise verweisen auf konkrete Betriebsvorgaben aus der Vielzahl von Betriebsvorgaben 76, die in dem Generator-Datenspeicher 72 gespeichert sind. Diese Betriebsvorgaben können Steuervorgaben, nämlich beispielsweise einzelne Steuerbefehle, zusammengesetzte Steueranweisungen oder Parameterwerte sein. Die durch die parametrisierten Verweise in der Datenstruktur 78 aufgerufenen Betriebsvorgaben 76 steuern den Betrieb des Elektrochirurgie-Generators 12 in dem jeweiligen Betriebsmodus in Kombination mit dem Betriebsprogramm 74. Die Betriebsvorgaben können auch Steuerbefehle sein, die den Aufruf anderer Verweise in der Datenstruktur 78 bewirken. Allerdings ist dies nur innerhalb der Daten - also innerhalb derjenigen Verweise - möglich, die zu einem jeweiligen Betriebsmodus gehören.

Die parametrisierten Verweise sind vorzugsweise durch Hexadezimalzahlen repräsentiert, die zusammen strukturierte Daten eines Datensatzes 80 bilden. Ein Datensatz gehört zu einem Betriebsmodus und kann zum Beispiel folgendes enthalten:

| | | |
|---|---|---|
| *006F* | **11** | |
| *0070* | **36** | 02 30 00 C8 00 |
| *0076* | **12** | |
| *0077* | **0F** | 04 64 00 |
| *0078* | **56** | |
| *007C* | **4B** | 03 02 |
| *007F* | **13** | |

Die kursiv dargestellten Zahlen sind beim Auslesen des Datensatzes 80 generierte Zeilennummern und sind nicht in dem Datenspeicher 26 des elektrochirurgischen Instrument 14 gespeichert, sondern werden von dem Betriebsprogramm entsprechend der Reihenfolge der Verweise in einem jeweiligen Datensatz selbst erzeugt. Die Zeilennummern ergeben sich somit aus der Reihenfolge der Verweise (also deren Struktur) in einem jeweiligen Datensatz. Die im Beispiel als kursiv dargestellte Zahlen dargestellten Zeilennummern sind einfach aufsteigende Zahlen entsprechend der Länge der strukturierten Daten. Die fett dargestellten Zahlen dienen als Verweise (oder Zeiger bzw. Pointer), die jeweils auf eine konkrete Betriebsvorgabe 76 im Generator-Datenspeicher 72 verweisen, und zwar in dem dargestellten Beispiel auf Steuerbefehle. So verweist z.B. "11" auf den Steuerbefehl "IF", "36" auf einen Vergleich, der durch die nachfolgenden, zugeordneten Zahlen "02 30 00 C8 00" spezifiziert wird, "12" auf den Steuerbefehl "THEN", "0F" auf einen Steuerbefehl zum Setzen eines durch die nachfolgenden, zugeordneten Zahlen "04 64 00" spezifizierten Parameterwertes und "56" auf den Steuerbefehl "ELSE".

Aus der jeweiligen mittels parametrisiertem Verweis aufgerufenen Betriebsvorgabe 76 ergibt sich auch, welche Zusatzinformationen (wie z.B. "02 30 00 C8 00" in dem obigen Beispiel) noch relevant sind.

Das Betriebsprogramm 74 kann das vorstehend dargestellte Beispiel wie folgt lesen und übersetzen:
**11** - if-> keine Zusatzinfos erforderlich
**36** - Vergleich -> 5 Zeichen an Zusatzinformationen erforderlich (02 = 1 Zeichen Vergleichsart, 30 00 = 2 Zeichen Zahl 1, C8 00 = 2 Zeichen Zahl 2)
**12** - then -> keine Zusatzinfos erforderlich
**0F** - Ausgangswert setzen -> 3 Zeichen Zusatzinformationen (04 = 1 Zeichen Welcher Ausgangswert; 64 00 = 2 Zeichen Setzwert)

Übersetzt kann dies bedeuten "*Vergleiche, ob Zahl 1 (3000) größer ist, als Zahl 2 (C8 00). Wenn das Ergebnis TRUE ist, dann setze Spannung auf 100 V (64 00), ansonsten* ..."

Die in dem Beispiel als Hexadezimalzahlen dargestellten Binärzahlen (und hier verkürzt als "Hexadezimalzahlen" bezeichneten Zahlen) in einem jeweiligen Datensatz 80 repräsentieren somit zum einen parametrisierte Verweise, auf deren Basis das Betriebsprogramm 74 auf konkrete Betriebsvorgaben 76 in dem Generator-Datenspeicher 72 zugreifen kann. Die in einem Datensatz strukturiert (geordnet) abgelegten Hexadezimalzahlen repräsentieren die parametrisierten Verweise auf in dem Generator-Datenspeicher 72 gespeicherte Betriebsvorgaben wie z.B. Steuerbefehle und Parameter, denen aufgrund der Struktur des Datensatzes Zeilennummern zugeordnet werden können, die selbst nicht explizit in einem Datensatz 80 gespeichert sein müssen, sondern von dem Betriebsprogramm 74 beim Einlesen eines jeweiligen Datensatzes 80 erzeugt werden können.

Die in einem jeweiligen Datensatz 80 gespeicherten und in dem Beispiel als Hexadezimalzahlen dargestellten Binärzahlen dienen als Pointer (oder Zeiger), die jeweils auf eine konkrete Betriebsvorgabe 76 in dem Generator-Datenspeicher 72 des Elektrochirurgie-Generators 12 verweisen, so dass die entsprechende Hexadezimalzahl mit einer entsprechenden Betriebsvorgabe 76 in dem Generator-Datenspeicher 72 des Elektrochirurgie-Generators 12 verknüpft ist. Diese Hexadezimalzahlen werden somit zur Bezeichnung eines entsprechenden, eine Betriebsvorgabe 76 repräsentierenden Speichereintrags in dem Generator-Datenspeicher 76 des Elektrochirurgie-Generators 12 verwendet. Diese Hexadezimalzahlen sind somit eine Art Pointer, um das Betriebsprogramm zu Betriebsvorgaben 76 in dem Generator-Datenspeicher 72 des Elektrochirurgie-Generators 12 zu leiten, wo das Betriebsprogramm jeweils eine Betriebsvorgabe für das Betriebsprogramm 74 während seiner Ausführung abrufen kann. Der Aufruf der Verweise erfolgt durch das Betriebsprogramm gesteuert und führt dazu, dass der Prozessor 70 die Speichereinträge in dem Generator-Datenspeicher 72 abruft, auf die die Verweise verweisen. Dabei sind auch Sprünge innerhalb der Verweise möglich, die durch ihre Sequenz oder Zeilennummern identifiziert sind. Die strukturierten Daten in einem Datensatz 80 können so über die ihnen entsprechenden Speichereinträge in dem Generator-Datenspeicher 72 in Betriebsvorgaben für das Betriebsprogramm übersetzt werden.

In dem vorstehend beispielhaft wiedergegebenen Datensatz stellen die Einträge in der ersten Spalte (*"006F, 0070, 0076..."*) Zeilennummern dar. Die Zeilennummernsind nicht in dem Instrumenten-Datenspeicher 26 des elektrochirurgischen Instrument 14 gespeichert, sondern werden von dem Betriebsprogramm 74 selbst erzeugt, da die Zeilennummerneinfach aufsteigende Zahlen entsprechend der Länge der strukturierten Daten sind:
*0070* 36 02 30 00 C8 00 (Länge der Daten = 6, d.h. nächste Zeilennummer ist *0076*)
*0076* 12 (Länge der Daten = 1, d. h. nächste Zeilennummerist *0077*)

Die Einträge in jeder Zeile ("**11**, **36** 02 30 00 C8 00, **12**...") verweisen auf Betriebsvorgaben 76 in dem Generator-Datenspeicher 72. Der durch 11 bezeichnete Eintrag in dem Generator-Datenspeicher 72 kann zum Beispiel eine "IF"-Anweisung sein, während der durch 12 bezeichnete Eintrag eine "THEN"-Anweisung sein kann. "IF"-Anweisung und "THEN"-Anweisung sind jeweils eine Betriebsvorgabe. Die in den strukturierten Daten des abgebildeten Datensatzes zwischen 11 und 12 stehenden Hexadezimalzahlen "**36** 02 30 00 C8 00" können aus den dazugehörigen Speichereinträgen in dem Generator-Datenspeicher 72 in eine Steueranweisung übersetzt werden, wie zum Beispiel *"Vergleiche den letzten abgelesenen Wert der Spannung (30 00) mit der Zahl 200 (C800), und wenn die Spannung kleiner oder gleich 200 ist, ist das Ergebnis "TRUE", andernfalls ist das Ergebnis "FALSE"".* Wenn das Betriebsprogramm 74 an der (vom Betriebsprogramm erzeugten) Adresse *"0077'* die Speichereinträge für die Zeichenfolge **"0F** 04 64 00" aus dem Generator-Datenspeicher 72 abruft, könnte dies eine Einstellung für den Elektrochirurgie-Generator 12 bezeichnen; die Einstellung kann z.B. sein, dass ein Maximalwert für die Ausgangsgleichspannung (0F 04) auf 100V (64 00) eingestellt ist.

Ein wichtiger Aspekt ist die Ausgabe von Benachrichtigungen an einen Benutzer über den Lautsprecher 94 oder die Anzeige 96. Auch hier hilft die beschriebene Struktur des Elektrochirurgie-Generators 12, indem Signalisierungsanweisungen repräsentierenden Daten in dem Instrumenten-Datenspeicher 26 des elektrochirurgischen Instruments 14 gespeichert sein können, und damit nicht in dem Elektrochirurgie-Generator 12 hinterlegt werden müssen.

Das erfindungsgemäße Speichern von Signalisierungsanweisungen repräsentierenden Daten in dem Instrumenten-Datenspeicher 26 des elektrochirurgischen Instruments 14 schafft insbesondere die Möglichkeit, Aktivierungstöne und andere akustische Signale flexibel über das elektrochirurgische Instrument 14 zu definieren. Praktisch wird beispielsweise eine Bytesequenz in den strukturierten Daten 80 in dem Instrumenten-Datenspeicher 26 - also beispielsweise in dem EEPROM des elektrochirurgischen Instruments 14 - abgelegt. Die Bytesequenz definiert beispielsweise den Aktivierungston oder andere akustische Signale. Folgende Möglichkeiten können unter anderem implementiert werden:
1. Abspielen einer im Elektrochirurgie-Generator 12 definierten Audiodatei (z. B. ActivationCut.wav)
2. Abspielen einer im Elektrochirurgie-Generator 12 definierten Audiodatei mit einem definierten Pulse-Pause- Verhältnis (z. B. ActivationCut.wav mit einer Periodendauer von 500 ms einer Pausezeit von 100 ms)
3. Abspielen einer definierten Frequenz über den im Elektrochirurgie-Generator implementierten Frequenzgenerator 92 (z. B. 500 Hz)
4. Abspielen einer definierten Frequenz über den im Elektrochirurgie-Generator implementierten Frequenzgenerator 92 mit einem definierten Pulse-Pause-Verhältnis (z. B. 500 Hz mit einer Periodendauer von 500 ms und einer Pausezeit von 100 ms)
5. Abspielen eines Frequenzgemisches über den im Elektrochirurgie-Generator 12 implementierten Frequenzgenerator 92 (z. B. 500 Hz zusammen mit 1000 Hz und 2000 Hz)
6. Abspielen eines Frequenzgemisches über den im Elektrochirurgie-Generator 12 implementierten Frequenzgenerator 92 mit einem definierten Pulse-Pause-Verhältnis (z. B. 500 Hz zusammen mit 1000 Hz und 2000 Hz mit einer Periodendauer von 500 ms und einer Pausezeit von 100 ms)
7. Änderung des Aktivierungstons in Abhängigkeit des Betriebsmodus (z. B. ein Aktivierungston A unter normalen Bedingungen und Aktivierungston B, wenn ein gewisser Widerstandswert erreicht ist).

Daraus ergibt sich eine sehr große Flexibilität, so dass mit Hilfe der Programmierschnittstelle 82 und den Datensätzen 80 in der Datenstruktur 78 auch sehr komplexe Tonsequenzen auf dem elektrochirurgischen Instrument 14 hinterlegt werden können.

In Bezug auf die Ausgabe akustischer Signale an einen Benutzer kann die Datenstruktur 78 - oder genauer: ein Datensatz in der Datenstruktur - zum Umsetzen der vorgenannten Beispiele folgende Einträge enthalten:
1) Abspielen einer im Generator-Datenspeicher 72 des Elektrochirurgie-Generators 12 gespeicherten Audiodatei
*Set Activation Sound File (Start. 3, 0 ms, 0 ms)*

| | | |
|---|---|---|
| *0048* | **46** | 00 03 00 00 00 00 00 |

2) Abspielen einer im Generator-Datenspeicher 72 des Elektrochirurgie-Generators 12 gespeicherten Audiodatei mit einem definierten Pulse-Pause- Verhältnis
*Set Activation Sound File(Start, 3, 500 ms, 100 ms)*

| | | |
|---|---|---|
| *0048* | **46** | 00 03 00 F4 01 64 00 |

3) Abspielen einer definierten Frequenz über den im Elektrochirurgie-Generator 12 implementierten Frequenzgenerator 92
*Set Activation Sound Frequency(Play, 500 Hz, 0 ms, 0 ms)*

| | | |
|---|---|---|
| *0048* | **47** | 00 F4 01 00 00 00 00 |

4) Abspielen einer definierten Frequenz über den im Elektrochirurgie-Generator 12 implementierten Frequenzgenerator 92 mit einem definierten Pulse-Pause-Verhältnis
*Set Activation Sound Frequency(Play, 500 Hz, 500 ms, 100 ms)*

| | | |
|---|---|---|
| *0048* | **47** | 00 F4 01 F4 01 64 00 |

5) Abspielen eines Frequenzgemisches über den im Elektrochirurgie-Generator 12 implementierten Frequenzgenerator 92
*Buffer(Initialize, activationTones_000, 3)*
*Buffer(Set Value Directly, activationTones_000. 0, 500)*
*Buffer(Set Value Directly, activationTones_000,* 1, *1000)*
*Buffer(Set Value Directly, activationTones_000, 2, 2000)*
*Set Activation Sound Multi Frequency(Play, activationTones_000, 0 ms, 0 ms)*

| | | |
|---|---|---|
| *0048* | **58** | 08 00 03 OD |
| *004D* | **58** | 30 00 GO 00 F4 01 |
| *0054* | **58** | 30 00 01 00 E8 03 |
| *005B* | **58** | 30 00 02 00 DO 07 |
| *0062* | **57** | 03 00 00 00 00 OD |

6) Abspielen eines Frequenzgemisches über den im Elektrochirurgie-Generator 12 implementierten Frequenzgenerator 92 mit einem definierten Pulse-Pause-Verhältnis
*Buffer(Initialize, activationTones_000, 3)*
*Buffer(Set Value Directly. activationTones_000, 0, 500)*
*Buffer(Set Value Directly. activationTones_000,* 1, *1000)*
*Buffer(Set Value Directly. activationTones_000, 2, 2000)*
*Set Activation Sound Mufti Frequency(Play, activationTones_000, 500 ms, 100 ms)*

| | | |
|---|---|---|
| *0048* | **58** | 03 00 03 00 |
| *004D* | **58** | 30 00 00 00 F4 01 |
| *0054* | **58** | 30 00 01 00 E8 03 |
| *0058* | **58** | 30 00 02 00 DO 07 |
| *0062* | **57** | 03 00 F4 01 64 00 |

7) Abspielen eines Aktivierungstons in Abhängigkeit des Betriebsmodus

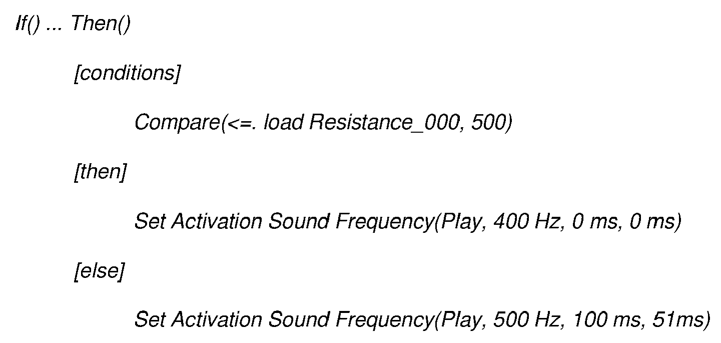

| | | |
|---|---|---|
| *004E* | **11** | |
| *004F* | **36** | 02 30 00 F4 01 |
| *0055* | **12** | |
| *0056* | **47** | 00 90 01 00 00 00 00 |
| *005E* | **56** | |
| *005F* | **47** | 00 F4 01 64 00 32 00 |
| *0067* | **13** | |

In Bezug auf Anzeigen von Text in einem je nach Anzeigemöglichkeit unterschiedlichen Anzeigeformat kann ein Benachrichtigungstext durch einen Zeichenstring repräsentiert sein, der zusätzlich Sonderzeichen oder nichtdruckbare Zeichen (Steuerzeichen) enthält, die insbesondere ein Kürzen eines Benachrichtigungstextes durch den von dem Betriebsprogramm 74, die Betriebsvorgaben 76 und die strukturierten Daten gesteuerten Prozessor 70 vorgeben, falls eine Anzeige 96 dies erfordert.

Dies macht es möglich, dass ein elektrochirurgisches Instrument 14 an verschiedenen Elektrochirurgie-Generatoren 12 betrieben werden kann, die unterschiedliche Anzeigen aufweisen. So kann auf geeigneten Anzeigen ein längerer Benachrichtigungstext angezeigt werden, als auf kleineren Anzeigen.

Der Zeichenstring bewirkt eine Flexibilität des anzuzeigenden Benachrichtigungstextes dadurch, dass der anzuzeigende Text auf dem elektrochirurgischen Instrument 14 zusammen mit wenigstens einer Kürzungsanweisung gespeichert ist. Als Kürzungsanweisung dienen beispielsweise in den Zeichenstring eingefügte Sonderzeichen oder nichtdruckbare Zeichen (hier auch als Steuerzeichen bezeichnet), die nach einem vordefinierten Regelwerk von dem Prozessor 70 für notwendige Kürzungen des anzuzeigenden Textes genutzt werden können. Es sind sowohl Kürzungen am Ende als auch am Anfang und inmitten des Zeichenstrings möglich. Kombinierte Kürzungen sind möglich, prioritätsgesteuert.

Ein Zeichenstring kann somit neben dem anzuzeigenden Text (dem eigentlichen String) auch Steuerzeichen enthalten, die eine Anpassung des anzuzeigenden Textes im Falle der Widergabe auf einer jeweiligen Anzeige 96 des Elektrochirurgie-Generators 12 definieren.

Dies sei nachfolgend anhand einiger vereinfachter Beispiele erläutert.

Vereinfachtes Beispiel 1 (Kürzungen am Ende):
Herkömmlicher Betriebsmodus-Name: "*BiSoftCoag"*
Betriebsmodus-Name mit Kürzungsanweisung: *"Bi#Soft#Coag"*
Auf der Anzeige des Elektrochirurgie-Generators (12) würde bei ausreichendem Platzangebot angezeigt werden: *"BiSoftCoag"*
Auf der Anzeige des Elektrochirurgie-Generators (12) würde bei verringertem Platzangebot angezeigt werden: *"BiSoft"*
Auf der Anzeige des Elektrochirurgie-Generators (12) würde bei sehr geringem Platzangebot angezeigt werden: *"Bi"*

Das Sonderzeiechen "#" wird also nie angezeigt und an den Stelen, an denen das Sonderziechen "#" steht, kann der anzuzeigende Zeichenstring gekürzt werden.

Vereinfachtes Beispiel 2 (Kürzungen an anderen Stellen):
Herkömmlicher Betriebsmodus-Name: *"PK SoftCoag"*
Betriebsmodus-Name mit Kürzungsanweisung: *"{PK}SoftCoag"*
Auf der Anzeige des Elektrochirurgie-Generators (12) würde bei ausreichendem Platzangebot angezeigt werden: *"PK SoftCoag"*
Auf der Anzeige des Elektrochirurgie-Generators (12) würde bei verringertem Platzangebot angezeigt werden: *"SoftCoag"*

Der Zeichenstring in den geschweiften Klammern wird also ggf. nicht angezeigt.

Vereinfachtes Beispiel 3 (Kombinierte Kürzungen):
Herkömmlicher Betriebsmodus-Name: *"PK SoftCoag"*
Betriebsmodus-Name mit Kürzungsanweisung: *"{PK}Soft#Coag"*
Auf der Anzeige des Elektrochirurgie-Generators (12) würde bei ausreichendem Platzangebot angezeigt werden: *"PK SoftCoag"*
Auf der Anzeige des Elektrochirurgie-Generators (12) würde bei verringertem Platzangebot angezeigt werden: *"PK Soft"*
Auf der Anzeige des Elektrochirurgie-Generators (12) würde bei sehr geringem Platzangebot angezeigt werden: *"Soft"*

Das Beispiel 3 zeigt, wie die in den Beispielen 1 und 2 dargestellten Maßnahmen zum Kürzen anzuzeigender Zeichenstrings miteinander kombiniert werden können.

Mit Hilfe der Signalisierungs- und Anzeigemittel in Form des Lautsprechers 94 nebst Tongenerator 92 und der Anzeige 96 ist es möglich einen Benutzer über Betriebszustände, Fehler etc. zu benachrichtigen. Die derartigen Benachrichtigungen definierenden Steueranweisungen sind durch Daten repräsentiert, die in dem Datenspeicher 26 des elektrochirurgischen Instruments 14 gespeichert sind. Benachrichtigungs- und Fehlerzustände sowie das Verhalten dieser Benachrichtigungen müssen somit nicht schon zum Zeitpunkt der Entwicklung des Elektrochirurgie-Generators bekannt sein. Auch ist eine nachträgliche Anpassung der Benachrichtigungs- und Fehlerzustände sowie das Verhalten dieser Benachrichtigungen leicht über ein Softwareupdate des elektrochirurgischen Instruments möglich. Sogar eine "live"-Optimierung der Benachrichtigungs- und Fehlerzustände sowie das Verhalten dieser Benachrichtigungen während Usability-Tests ist möglich.

Das erfindungsgemäße Speichern von Signalisierungsanweisungen repräsentierenden Daten in dem Datenspeicher 26 des elektrochirurgischen Instruments 14 schafft außerdem die Möglichkeit, Benachrichtigungen und Fehlermeldungen sowie das Verhaltens dieser Benachrichtigungen flexibel auf dem elektrochirurgischen Instrument 14 zu definieren. Praktisch wird beispielsweise eine Bytesequenz in den strukturierten Daten 80 in dem Instrumenten-Datenspeicher 26 des elektrochirurgischen Instruments 14 - also beispielsweise in dem im EEPROM - abgelegt. Die Bytesequenz definiert beispielsweise die Bedingungen und Eigenschaften für eine bestimmte Benachrichtigung oder Fehlermeldung.

Die nachfolgend beispielhaft erläuterten Benachrichtigungs- und Fehlermeldungstypen können auf dem elektrochirurgischen Instrument definiert werden.

### 1. Anzeigen eines Infotextes

- flexible Auslösebedingung
- flexible Soundgestaltung (lautlos, Standard-Info-Sound, selbst-definierter Sound)
- flexibles Hochfrequenz/Ultraschall-Handling (mit Deaktivierung von HF/US, ohne Deaktivierung von Hochfrequenz/Ultraschall)

### 2. Anzeigen eines Low-Priority-Fehlers mit Standard-Low-Priority-Sound

- flexible Auslösebedingung
- flexibles Hochfrequenz/Ultraschall-Handling (mit Deaktivierung von Hochfrequenz/Ultraschall, ohne Deaktivierung von Hochfrequenz oder Ultraschall)

### 3. Anzeigen eines Medium-Priority-Fehlers mit Standard-Medium-Priority-Sound

- flexible Auslösebedingung
- flexibles Hochfrequenz -Handling (mit Deaktivierung von Hochfrequenz/Ultraschall, ohne Deaktivierung von Hochfrequenz/Ultraschall)

### 4. Anzeigen eines High-Priority-Fehlers mit Standard-High-Priority-Sound

Folgende Auslösebedingungen sind unter anderem möglich:
1. Beim Anschließen eines elektrochirurgischen Instruments 14 an den Elektrochirurgie-Generator 12
2. Im "Idle"-Zustand des Elektrochirurgie-Generators 12 (z. B. nach Ablauf einer definierten Zeitdauer seit dem Anschließen eines elektrochirurgischen Instruments 14)
3. Kurz vor der Ausgabe von Hochfrequenz/Ultraschall (z. B. Verhinderung der Aktivierung, falls das elektrochirurgische Instrument 14 abgelaufen ist)
4. Kurz nach der Ausgabe von Hochfrequenz/Ultraschall (z. B. Mitteilung, dass die Anwendung noch nicht abgeschlossen ist)
5. Während der Aktivierung in Abhängigkeit des Betriebsmodus, z.B.
   - In Abhängigkeit der Gewebeimpedanz
   - In Abhängigkeit der Aktivierungsdauer
   - Beim Auftreten bestimmter Ereignisse (z. B. Funken)

Es ist auch möglich, dass auch die angezeigten Texte mehrsprachig auf dem elektrochirurgischen Instrument - d.h. in dem Instrumenten-Datenspeicher 26 - hinterlegt sind.

Die folgenden Implementierungsbeispiele illustrieren dies:
A) Beispiele für verschiedene Benachrichtigungstypen
Lautloser Infotext ohne Hochfrequenz/Ultraschall-Deaktivierung
*Generate Error(UInt 16. 666)*

| | | |
|---|---|---|
| *0068* | **4A** | 00 9A 02 |

Lautloser Infotext mit Hochfrequenz/Ultraschall-Deaktivierung
*Generate Error(UInt 16. 666)*
*Stop Activation°*

| | | |
|---|---|---|
| *0068* | **4A** | 00 9A 02 |
| *006C* | **53** | |

Lautloser Infotext mit Hochfrequenz/Ultraschall-Deaktivierung und eigener Soundgestaltung
*Generate Error(UInt 16. 666)*
*Set Activation Sound Frequency(Play, 750 Hz. 100 ms, 50 ms)*
*Busy Wait(Time, 1000)*
*Stop Activation°*

| | | |
|---|---|---|
| *0068* | **4A** | 00 9A 02 |
| *006C* | **47** | 00 EE 02 64 00 32 00 |
| *0074* | **48** | 00 E8 03 |
| *0078* | **53** | |

Low-Priority-Error ohne Hochfrequenz/Ultraschall-Deaktivierung
*Generate Error(UInt 16. 676)*

| | | |
|---|---|---|
| *0068* | **4A** | 00 A4 02 |

Low-Priority-Error mit Hochfrequenz/Ultraschall-Deaktivierung
*Generate Error(UInt16, 676)*
*Stop Activation°*

| | | |
|---|---|---|
| *0068* | **4A** | 00 A4 02 |
| *006C* | **53** | |

Medium-Priority-Error ohne Hochfrequenz/Ultraschall-Deaktivierung
Generate Error(Ulnt 16, 188)

| | | |
|---|---|---|
| *0068* | **4A** | 00 BC 00 |

Medium-Priority-Error mit Hochfrequenz/Ultraschall-Deaktivierung
*Generate Error(UInt16, 188)*
*Stop Activation°*

| | | |
|---|---|---|
| *0068* | **4A** | 00 BC 00 |
| *006C* | **53** | |

High-Priority-Error
*Generate Error(UInt16. 14)*

| | | |
|---|---|---|
| *0068* | **4A** | 00 OE 00 |

B) Beispiele für Auslösebedingungen
Beim Anschließen eines elektrochirurgischen Instruments
*errorOnConnecti_000*
*[options]*
*Aditional Execution: On Instrument Connection*
*[parameters]*
*[content]*
*Generate Error(UInt16, 1)*

| | | |
|---|---|---|
| *001D* | **00** | 0B 00 |
| *0020* | **01** | 00 |
| *0022* | **04** | |
| *0023* | **00** | 00 |
| *0025* | **04** | 00 |
| *0027* | **4A** | 00 01 00 |

Im "Idle"-Zustand des Elektrochirurgie-Generators (z. B. nach Ablauf einer definierten Zeitdauer seit dem Anschließen eines elektrochirurgischen Instruments)

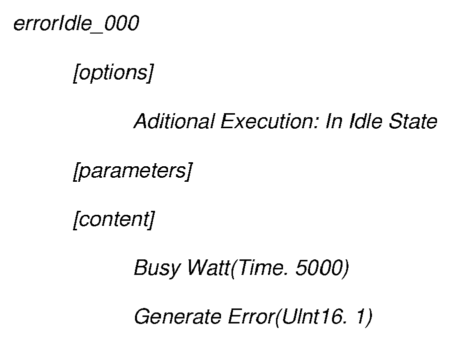

| | | |
|---|---|---|
| *001D* | **00** | 0F 00 |
| *0020* | **01** | 00 |
| *0022* | **01** | |
| *0023* | **00** | 00 |
| *0025* | **08** | 00 |
| *0027* | **48** | 00 88 13 |

Kurz vor der Ausgabe von Hochfrequenz oder Ultraschall

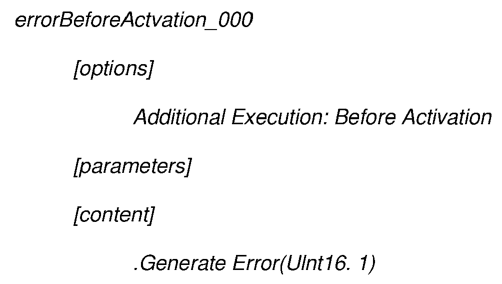

| | | |
|---|---|---|
| *0010* | **00** | 0B 00 |
| *0020* | **01** | 00 |
| *0022* | **02** | |
| *0023* | **00** | 00 |
| *0025* | **04** | 00 |
| *0027* | **4A** | 00 01 00 |

Kurz nach Ausgabe von Hochfrequenz oder Ultraschall

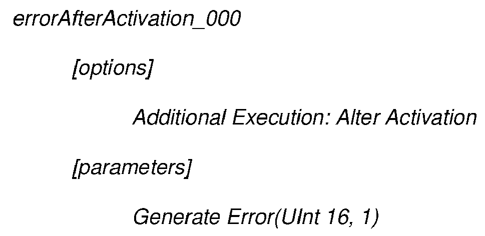

| | | |
|---|---|---|
| *001D* | **00** | 0B 00 |
| *0020* | **01** | 00 |
| *0022* | **03** | |
| *0023* | **00** | 00 |
| *0025* | **04** | 00 |
| *0027* | **4A** | 00 01 00 |

Während der Aktivierung in Abhängigkeit der Gewebeimpedanz

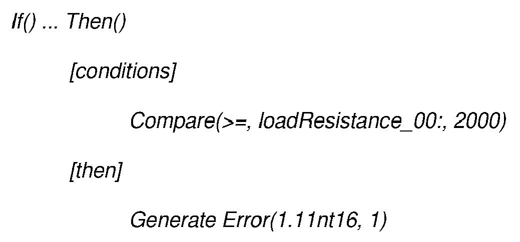

| | | |
|---|---|---|
| *007C* | **11** | |
| *0070* | **02** | 1000 DO 07 |
| *0083* | **12** | |
| *0084* | **4A** | 00 01 00 |
| *0088* | **13** | |

Der Betrieb des Elektrochirurgie-Generators 12 in einem jeweiligen Betriebsmodus hängt somit zum einen von dem in dem Generator-Datenspeicher 72 gespeicherten Betriebsprogramm 74 ab. Darüber hinaus hängt das Betriebsverhalten des Elektrochirurgie-Generators 12 in einem jeweiligen Betriebsmodus aber auch von dem für einen jeweiligen Betriebsmodus aufgerufenen Datensatz 80 in der Datenstruktur 78 ab sowie von den ebenfalls im dem Generator-Datenspeicher 72 gespeicherten Betriebsvorgaben 76 und insbesondere auch von den in dem Instrumenten-Datenspeicher 26 des elektrochirurgischen Instruments 14 gespeicherten Daten, die Signalisierungsanweisungen repräsentieren. Das Betriebsverhalten schließt dabei sowohl die Abgabe von Ausgangswechselspannung an das elektrochirurgische Instrument 14 ein, als auch das Anzeigen von Text über die Anzeige 96 oder das Ausgeben von akustischen Signalen über den Lautsprecher 94.

Der Vorteil eines solchen Elektrochirurgie-Generators 12 ist, dass neue Betriebsmodi einschließlich der zugehörigen Benachrichtigungen für einen Benutzer leicht durch Erzeugen neuer Datensätze 80 in der Datenstruktur 78 definiert werden können und beispielsweise ein einzelner Betriebsmodus allein durch Ändern des entsprechenden Datensatzes 80 in der Datenstruktur 78 geändert werden kann, ohne dass das Betriebsprogramm 74 in dem Generator-Datenspeicher 72 oder die Betriebsvorgaben 72 in dem Generator-Datenspeicher 72 geändert werden müssen. Insbesondere können Signalisierungsanweisungen für ein elektrochirurgisches Instrument 14 leicht durch Daten in dem Datenspeicher 26 des elektrochirurgischen Instruments 14 definiert werden.

Benachrichtigungs- und Fehlerzustände sowie das Verhalten dieser Benachrichtigungen müssen somit nicht schon zum Zeitpunkt der Entwicklung des Elektrochirurgie-Generators bekannt sein. Auch ist eine nachträgliche Anpassung der Benachrichtigungs- und Fehlerzustände sowie das Verhalten dieser Benachrichtigungen leicht über ein Softwareupdate des elektrochirurgischen Instruments möglich. Sogar eine "live"-Optimierung der Benachrichtigungs- und Fehlerzustände sowie das Verhalten dieser Benachrichtigungen während Usability-Tests ist möglich.

Auf der anderen Seite können globale Parameter, wie beispielsweise die möglichen zur Verfügung stehenden Steueranweisungen oder vom Elektrochirurgie-Generator abhängigen Betriebsparameter, wie dessen maximale Ausgangswechselspannung oder eine minimal zulässige Ausgangsgleichspannung als Betriebsvorgaben 76 in dem Generator-Datenspeicher 76 hinterlegt sein und dort auch gegebenenfalls zentral für alle möglichen Betriebsmodi zugleich geändert werden.

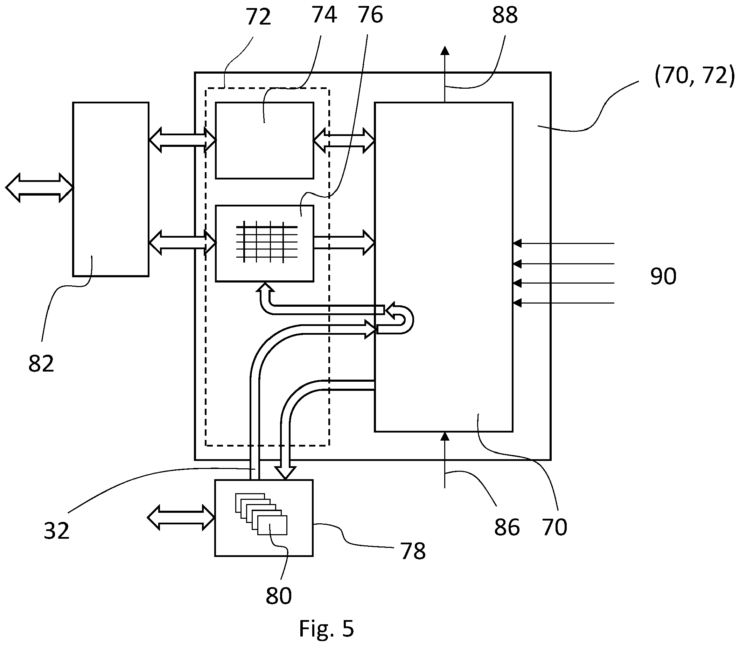

Ein weiterer Vorteil des Aufbaus des Elektrochirurgie-Generators 12 ist, dass ein Datensatz 80, dessen strukturierte Daten indirekt einen für das elektrochirurgische Instrument 14 geeigneten Betriebsmodus definieren, auch in einem elektrochirurgischen Instrument 14 hinterlegt sein kann; siehe Figur 5. Konkret kann der Instrumenten-Datenspeicher 26 des elektrochirurgischen Instruments 14 einen Datensatz mit strukturierten Daten enthalten, die zu der Datenstruktur 78 in dem Generator-Datenspeicher 72 kompatibel sind und genau wie andere strukturierte Daten in der Datenstruktur 78 durch entsprechende Verweise auf Betriebsvorgaben 76 in dem Generator-Datenspeicher 72 indirekt einen jeweiligen Betriebsmodus definieren. Dies ist insbesondere im Hinblick auf akustische oder optische Signale zum Benachrichtigen eines Benutzers über Betriebszustände oder Fehler etc. von großem Vorteil, weil derartige Signale definierende Steueranweisungen für ein individuelles elektrochirurgisches Instrument 14 durch Daten repräsentiert sein können, die in dem Instrumenten-Datenspeicher 26 dieses elektrochirurgischen Instruments 14 passend zum jeweiligen individuellen elektrochirurgischen Instrument 14 gespeichert sein können.

Der Elektrochirurgie-Generator 12 ist daher so konfiguriert, dass er beim Anschließen eines elektrochirurgischen Instruments 14 jeweils - soweit vorhanden - zunächst dessen Instrumenten-Datenspeicher 26 ausliest und die strukturierten Daten aus dem in dem Instrumenten-Datenspeicher 26 gespeicherten Datensatz in die Datenstruktur 78 im Generator-Datenspeicher 72 einträgt. Auf diese Weise steht dem Elektrochirurgie-Generator 12 dann im Betrieb ein genau auf das jeweilige elektrochirurgische Instrument 14 zugeschnittener Betriebsmodus zur Verfügung.

Um einen Zugriff auf die Inhalte des Instrumenten-Datenspeichers 26 zu erlauben, ist wenigstens die Datenleitung 22 mit einem zugehörigen Anschluss 18.3 vorgesehen. Alternativ oder zusätzlich kann auch eine drahtlose Schnittstelle wie beispielsweise eine Bluetooth Schnittstelle oder eine NFC Schnittstelle für den Zugriff auf die Inhalte des Instrumenten-Datenspeichers 26 vorgesehen sein.

Beim Übertragen der strukturierten Daten von dem Instrumenten-Datenspeicher 26 des elektrochirurgischen Instruments 14 in einem entsprechenden Datensatz in der Datenstruktur 78 des Elektrochirurgie-Generators 12 können ggf. die Zeilennummern passend zu dem Betriebsprogramm 74 des Elektrochirurgie-Generators 12 generiert werden.

Von großem Vorteil ist, dass der in dem Instrumenten-Datenspeicher 26 gespeicherte Datensatz nur strukturiert geordnete Verweise (Pointer auf weitere Speichereinträge in dem Generator-Datenspeicher 72) enthält, jedoch nicht unmittelbar irgendwelche Steueranweisungen oder Betriebsparameter für einen jeweiligen Betriebsmodus, da die Steueranweisungen und die Betriebsparameter in dem Generator-Datenspeicher 76 des Elektrochirurgie-Generators 12 zentral gespeichert sind.

Alternativ kann der Elektrochirurgie-Generator 12 auch so konfiguriert sein, dass er während des Betriebs - also während des Ablaufs des Betriebsprogramms - den Instrumenten-Datenspeicher 26 des elektrochirurgischen Instruments direkt 14 ausliest. Dies ist bei dem in Figur 5 dargestellten Beispiels der Fall. In diesem Fall ist es nicht nötig, dass die strukturierten Daten des Datensatzes in dem Instrumenten-Datenspeicher 26 zunächst in die Datenstruktur 78 auf dem Elektrochirurgie-Generator 12 übertragen werden. Die zu generierenden Zeilennummern müssen dann allerdings zu den entsprechenden Aufrufen in dem Betriebsprogramm und den Einträgen in dem Generator-Datenspeicher 72 passen.

Ein Vorteil eines Elektrochirurgie-Systems 10 der hier beschriebenen Art ist, dass verschiedene, den Betrieb des Elektrochirurgie-Generators 12 bestimmende Parameterwerte und Betriebsvorgaben unabhängig voneinander gepflegt werden können. So ist das in dem Generator-Datenspeicher 72 gespeicherte Betriebsprogramm 74 unabhängig von den Betriebsvorgaben 76 in dem Generator-Datenspeicher 72 gespeichert. Die Betriebsvorgaben 76 sind wiederum unabhängig von den strukturierten Daten in der Datenstruktur 78 gespeichert.

Vorzugsweise ist daher eine Programmierschnittstelle 82 vorgesehen, die vorzugsweise eine graphische Benutzeroberfläche bereitstellt, über einen Datensatz mit strukturierten Daten, die als eine Vielzahl von parametrisierten Verweisen ausgeführt sind, erstellt werden und in dem Instrumenten-Datenspeicher 26 des elektrochirurgischen Instruments 14 abgelegt werden kann.

Vorzugsweise ist die Programmierschnittstelle 82 so konfiguriert, dass sie unterschiedlichen Nutzern unterschiedliche Rechte einräumt. So können die Rechte für das Programmieren des Betriebsprogramms 74, das in dem Generator-Datenspeicher 72 gespeichert ist, für das Eingeben der Betriebsvorgaben 76, die ebenfalls in dem Generator-Datenspeicher 72 gespeichert sind und für die strukturierten Daten, die in der Datenstruktur 78 gespeichert sind, unterschiedlich vergeben werden. Insbesondere kann auf diese Weise sichergestellt werden, dass Änderungen an den Betriebsvorgaben 76 oder Änderungen an dem Betriebsprogramm 74 nur von Entwicklern vorgenommen werden können, die mit dem jeweiligen Elektrochirurgie-Generator 12 vertraut sind. Die Programmierung des Betriebsprogramms 74 und der Betriebsvorgaben 76 kann somit durch Entwickler erfolgen, die mit dem jeweiligen Elektrochirurgie-Generator 12 vertraut sind, während die Definition von Betriebsmodi für ein elektrochirurgisches Instrument 14 durch Schaffen eines entsprechenden Datensatzes mit strukturierten Daten durch einen mit dem elektrochirurgischen Instrument 14 vertrauten Entwickler erfolgen kann. Vorzugsweise werden von einem mit dem elektrochirurgischen Instrument 14 vertrauten Entwickler erzeugte Datensätze in dem Instrumenten-Datenspeicher 26 des entsprechenden elektrochirurgischen Instruments 14 gespeichert, während weitere Betriebsmodi auch direkt in der Datenstruktur 78 auf dem Elektrochirurgie-Generator 12 gespeichert werden können. Hierzu kann der Elektrochirurgie-Generator 12 beispielsweise eine USB-Programmierschnittstelle aufweisen. Für in dem Instrumenten-Datenspeicher 26 des entsprechenden elektrochirurgischen Instruments 14 gespeicherte strukturierte Daten steht entweder die Datenleitung 32 in dem Anschlusskabel 16 mit entsprechender Schnittstelle zur Verfügung - oder, alternativ oder zusätzlich - auch eine drahtlose Schnittstelle wie beispielsweise eine Bluetooth Schnittstelle oder eine NFC Schnittstelle. Die strukturierten Daten aus dem Datensatz in dem Instrumenten-Datenspeicher 26 des elektrochirurgischen Instruments 14 können dann beim Anschließen des elektrochirurgischen Instruments 14 in die Datenstruktur 78 auf dem Elektrochirurgie-Generator 12 übertragen werden.

Dies ist insbesondere in Bezug auf verschiedene elektrochirurgische Instrumente 14 von Bedeutung, da die elektrochirurgischen Instrumente 14 möglicherweise von anderen Entwicklern programmiert werden als der Elektrochirurgie-Generator 12. Die Entwickler des Elektrochirurgie-Generators 12 können alle für den Elektrochirurgie-Generator 12 wichtigen spezifischen Parameterwertedaten und Steuerbefehle - gegebenenfalls in Abhängigkeit von dem in dem Generator-Datenspeicher 72 gespeicherten Betriebsprogramm 74 - als Betriebsvorgaben 76 in dem Generator-Datenspeicher 72 ablegen. Solche Parameterwerte können beispielsweise maximal oder minimal zulässige Werte für die Ausgangsgleichspannung, die Ausgangswechselspannung, etc. sein.

Davon unabhängig, können Entwickler eines elektrochirurgischen Instruments 14 mithilfe der strukturierten Daten in dem Datensatz in dem Instrumenten-Datenspeicher 26 des elektrochirurgischen Geräts genau vorgeben, wie ein spezifischer Betriebsmodus für dieses elektrochirurgische Instrument 14 im Rahmen der durch das Betriebsprogramm 74 in dem Generator-Datenspeicher 72 und die Betriebsvorgaben 76 in dem Generator-Datenspeicher 72 definierten Grenzwerte ablaufen kann. Dabei müssen sich die Entwickler des elektrochirurgischen Instruments 14 keine weiteren Gedanken über die Vorgaben durch das Betriebsprogramm 74 und die Betriebsvorgaben 76 machen. Vielmehr können die Entwickler des elektrochirurgischen Instruments 14 diese für den jeweiligen Elektrochirurgie-Generator 12 gegebenen Vorgaben hinnehmen.

Den Entwicklern eines elektrochirurgischen Instruments 14 steht zum Definieren eines Betriebsmodus für das jeweilige elektrochirurgische Instrument 14 eine Programmierschnittstelle 82 zur Verfügung, über die ein Entwickler einen Betriebsmodus für ein jeweiliges elektrochirurgisches Instrument 14 vollständig definieren kann. Dies schließt beispielsweise alle Strom- und Spannungsparameterwerte ein sowie auch Zeitvorgaben und Übergangsbedingungen für den Betrieb des elektrochirurgischen Instruments. Auf diese Weise kann die Entwicklung des Betriebsmodus mithilfe eines einfach zu bedienenden Werkzeugs nahezu ohne Software-Entwicklungskenntnisse durch einen Entwickler für ein elektrochirurgisches Instrument erfolgen. Die Programmierschnittstelle 82 stellt dem Entwickler eine Reihe von Parametersätzen zur Verfügung, mit denen dieser verschiedene Phasen, zum Beispiel die Anschnittsphase, die Schneidphase, die Koagulationsphase, aber auch Kurzschluss- oder Leistungsüberwachungen für das jeweilige elektrochirurgische Instrument definieren kann. Ein zu der Programmierschnittstelle 82 gehörendes Entwicklungswerkzeug erzeugt aus den Vorgaben einen speicherplatzsparenden Satz strukturierter Daten, der einen Datensatz bildet, der in dem Datenspeicher 26 des elektrochirurgischen Instruments 14 nichtflüchtig hinterlegt werden kann.

Falls ein durch strukturierte Daten in einem Datensatz 80 definierter, neuer Betriebsmodus für ein elektrochirurgisches Instrument 14 auch eine Veränderung des Betriebsprogramms 74 in dem Generator-Datenspeicher 72 oder der Betriebsvorgaben 76 in dem Generator-Datenspeicher 72 erfordert, können derartige Veränderungen beispielsweise von einem mit dem Elektrochirurgie-Generator 12 vertrauten Entwickler über eine Programmierschnittstelle 82 vorgenommen werden. So kann sichergestellt werden, dass die einen Betriebsmodus definierten strukturierten Daten zu den Betriebsvorgaben 76 in dem Generator-Datenspeicher 72 und zu dem Betriebsprogramm 74 in dem Generator-Datenspeicher 72 kompatibel sind. Der Entwickler des elektrochirurgischen Instruments 14 kann sich diesbezüglich mit dem mit dem Elektrochirurgie-Generator 12 vertrauten Entwickler abstimmen. Im Ergebnis ist sichergestellt, dass ein nicht mit dem Elektrochirurgie-Generator 12 vertrauter Entwickler - z.B. ein Entwickler für ein elektrochirurgisches Instrument 14 - keine fehlerhaften Betriebsvorgaben 76 erstellt oder das Betriebsprogramm 74 nicht so verändert, dass es zu unbeabsichtigten Effekten oder Fehlern kommt.

Wird ein elektrochirurgisches Instrument 14 an den Elektrochirurgie-Generator 12 angeschlossen, liest der Elektrochirurgie-Generator 12 den Instrumenten-Datenspeicher 26 in dem elektrochirurgischen Instrument 14 aus und ruft die durch die in den strukturieren Daten enthaltenen Verweise bezeichneten Betriebsvorgaben 76 in dem Generator-Datenspeicher 72 auf, sodass die dort definierten Strom- und Spannungsverläufe inklusive aller Zeitbedingungen und sonstiger Bedingungen angewandt werden. Auf diese Weise sind reduzierte Entwicklungszeiten und -kosten möglich. Das elektrochirurgische Instrument 14 kann weitestgehend unabhängig von einem Elektrochirurgie-Generator 12 integriert werden. Auch sind kürzere Produkteinführungszeiten möglich, da Betriebsmodi auch nach Einführen eines Elektrochirurgie-Generators 12 entwickelt und fertiggestellt werden können. Optimierungen eines Betriebsmodus und neue Betriebsmodi können leicht durch aktualisierte oder neue elektrochirurgische Instrumente 14 eingeführt werden. Die Definition eines Betriebsmodus für ein elektrochirurgisches Instrument kann nahezu ohne Software-Entwicklungskenntnisse erfolgen.

Wenn das Elektrochirurgiesystem 10 mit einem an den Elektrochirurgie-Generator 12 angeschlossenen elektrochirurgischen Instrument 14 betrieben werden soll, steht der geeignete Betriebsmodus bereits nach Anschließen des elektrochirurgischen Instruments 14 zur Verfügung, weil die zugehörigen Datensätze 80 mit den strukturierten Daten von dem Instrumenten-Datenspeicher26 des elektrochirurgischen Instruments 14 ausgelesen werden können. Ein Nutzer muss daher nur noch beispielsweise einen Schalter 84 betätigen, um das elektrochirurgische Instrument 14 in dem geeigneten Betriebsmodus des Elektrochirurgie-Generators 12 zu betreiben. Einstellungen oder Programmierungen muss der Nutzer nach Anschließen des elektrochirurgischen Instruments 14 nicht mehr vornehmen.

Der Schalter 84 ist über eine Leitung 86 mit dem Prozessor 70 des Elektrochirurgie-Generators 12 verbunden, so dass mit Betätigen des Schalters 84 der Ablauf des in dem Generator-Datenspeicher 72 gespeicherten Betriebsprogramms 74 gestartet und unterbrochen werden kann. Der Schalter 84 kann ein Fußschalter sein, er kann aber auch ein Handschalter sein, der sich beispielsweise an dem elektrochirurgischen Handinstrument 14 befindet. Anstelle der Leitung 86 kann auch hier eine drahtlose Steuerverbindung vorgesehen sein.

Eine weitere Alternative zu einem Schalter 84 ist ein automatisches Starten des Betriebsprogramms 74, das ein Benutzer vorab aktivieren kann. In diesem Fall wird von dem elektrochirurgischen Instrument 14 zunächst eine kleine Messspannung ausgegeben, um mit deren Hilfe einen Gewebekontakt (Stromfluss) zu detektieren. Wird ein Gewebekontakt - also ein Stromfluss - detektiert, wird das eigentliche Betriebsprogramm 74 für das elektrochirurgische Instrument aufgerufen. Verschwindet der Gewebekontakt, wird das eigentliche Betriebsprogramm 74 für das elektrochirurgische Instrument beendet, und es wird wieder eine kleine Messspannung ausgegeben, um mit deren Hilfe einen erneuten Gewebekontakt zu detektieren.

Durch das Betriebsprogramm gesteuert, greift der Prozessor 70 während der Anwendung eines Betriebsmodus indirekt auf einzelne Betriebsvorgaben 76 in dem Generator-Datenspeicher 72 zu, indem zunächst ein Zugriff auf Verweise in der Datenstruktur 78 erfolgt und daraufhin ein Abruf derjenigen Betriebsvorgabe 76 oder Betriebsvorgaben 76 erfolgt, auf die ein jeweiliger Verweis verweist. In Bezug auf die Ausgabe von Benachrichtigungen an einen Benutzer greift der Prozessor 70 während der Anwendung eines Betriebsmodus indirekt beispielsweise auf einzelne Audiodateien in dem Generator-Datenspeicher 72 zu, indem zunächst ein Zugriff auf Signalisierungsanweisungen repräsentierende Daten in dem Instrumenten-Datenspeicher 26 erfolgt und daraufhin ein Abruf derjenigen Audiodatei und ein Ansprechen des Tongenerators 92 erfolgt, auf die durch die Signalisierungsanweisung vorgegeben ist.

Abhängig sowohl von dem Betriebsprogramm 74 als auch den Betriebsvorgaben 76 in dem Generator-Datenspeicher als auch von den Verweisen und Signalisierungsanweisungen in der Datenstruktur 78 sowie von Signalen 90, die von den Erfassungseinheiten 54, 56 und 58 stammen, generiert der Prozessor 70 Steuersignale 88 für die Generatorsteuereinheit 46.

### Bezugszeichen

- 10: Elektrochirurgiesystem
- 12: Elektrochirurgie-Generator
- 14: elektrochirurgisches Instrument
- 16: Anschlusskabel
- 18.1, 18.2: elektrischen Ausgänge
- 18.3: Anschluss
- 20: Schaft
- 20.1, 20.2: Ausgänge
- 22: aktive Elektrode
- 24: Handgriff
- 26: Instrumenten-Datenspeicher
- 28, 30: Versorgungsleitungen
- 32: Datenleitung
- 40: Hochspannungsnetzteil
- 42: Ausgang
- 44: Hochfrequenzteil
- 46: Ausgangstransformator
- 48: Generatorsteuereinheit
- 50: Kondensator
- 52: Synchronisierschaltung
- 54: Ausgangsstromerfassungseinheit
- 56: Ausgangswechselspannungserfassungseinheit
- 58: Ausgangsgleichspannungserfassungseinheit
- 60: Hochspannungsgleichrichterschaltung
- 62: Ausgangskondensator
- 64: Schalter
- 70: Prozessor
- 72: Generator-Datenspeicher
- 74: Betriebsprogramm
- 76: Betriebsvorgaben
- 78: Datenstruktur
- 80: Datensatz
- 84: Schalter
- 86: Leitung
- 88: Steuersignale der des Prozessors
- 90: Signale der Erfassungseinheiten
- 92: Tongenerator inkl. Frequenzgenerator
- 94: Lautsprecher
- 96: Anzeige für Text und/oder Symbole

## Patentansprüche

1. Nicht therapeutisches Verfahren zum Betreiben eines Elektrochirurgiesystems (10) mit einem elektrochirurgischen Instrument (14), das mindestens einen Instrumenten-Datenspeicher aufweist, und mit einem Elektrochirurgie-Generator (12), an den das elektrochirurgische Instrument (14) im Betrieb angeschlossen ist, wobei der Elektrochirurgie-Generator (12) über einen Prozessor (70) und mindestens einen mit dem Prozessor (70) verbundenen Generator-Datenspeicher (72) verfügt, wobei der Prozessor (70) zur Steuerung des Elektrochirurgie-Generators (12) ein in dem Generator-Datenspeicher (72) hinterlegtes Betriebsprogramm (74) ausführt
in dem Instrumenten-Datenspeicher des elektrochirurgischen Instruments ein Datensatz hinterlegt wird oder ist und
bei Inbetriebnahme oder im Betrieb des Elektrochirurgie-Generators (12) der im Instrumenten-Datenspeicher (72) des elektrochirurgischen Instruments (14) hinterlegte Datensatz von dem Elektrochirurgie-Generator ausgelesen wird
**dadurch gekennzeichnet, dass**
der Datensatz strukturierte Daten enthält, die als eine Vielzahl von parametrisierten Verweisen ausgeführt sind und auf im Generator-Datenspeicher (72) des Elektrochirurgie-Generators (12) hinterlegte Betriebsvorgaben (76) verweisen, die potentiell anzuwendenden Steuerbefehle umfassen und zusätzlich Betriebsparameterwerte umfassen können, und
die in den strukturierten Daten hinterlegten parametrisierten Verweise durch das Betriebsprogramm (74) gesteuert aufgerufen werden, um die im Elektrochirurgie-Generator (12) hinterlegten Steuerbefehle unter Berücksichtigung von in den strukturierten Daten gegebenenfalls vorliegenden Betriebsparametern auszuführen, so dass der Elektrochirurgie-Generator in Abhängigkeit des Betriebsprogramms, der in dem Generator-Datenspeicher hinterlegten Betriebsvorgaben und der in den strukturierten Daten enthaltenen parametrisierten Verweise auf einzelne der in dem Generator-Datenspeicher hinterlegten Steuerbefehle gesteuert wird.

2. Verfahren nach Anspruch 1, wobei der im Instrumenten-Datenspeicher (26) des elektrochirurgischen Instruments (14) hinterlegte Datensatz mit strukturierten Daten nach dem Auslesen durch den Elektrochirurgie-Generator in dessen Generator-Datenspeicher (74) abgelegt wird.

3. Verfahren nach Anspruch 2, wobei in dem Instrumenten-Datenspeicher (26) des elektrochirurgischen Instruments (14) hinterlegte strukturierte Daten beim Auslesen durch den Elektrochirurgie-Generator (12) oder nach dem Ablegen in dem Generator-Datenspeicher (72) des Elektrochirurgie-Generators (12) geändert werden.

4. Verfahren nach wenigstens einem der vorherigen Ansprüche, wobei die strukturierten Daten Signalisierungsanweisungen umfassen, die im Betrieb des Elektrochirurgie-Generators in konfigurations- und/oder betriebsstatusabhängige akustische und/oder optische Benachrichtigungen an einen Benutzer umgesetzt werden.

5. Verfahren nach wenigstens einem der vorherigen Ansprüche, wobei die strukturierten Daten Definitionen für Aktivierungstöne umfassen.

6. Verfahren nach wenigstens einem der vorherigen Ansprüche, wobei die strukturierten Daten Bedingungen und Eigenschaften für das Erzeugen und/oder Wiedergeben von Benachrichtigungen und/oder Fehlermeldungen umfassen.

7. Verfahren nach wenigstens einem der vorhergehenden Ansprüche, wobei die strukturierten Daten für Textnachrichten repräsentierende Daten Steuerzeichen umfassen, die die Widergabe von Textnachrichten auf unterschiedlichen Anzeigen definieren und Textnachrichten im Betrieb des Elektrochirurgie-Generators (12) in Abhängigkeit der Steuerzeichen und einer an dem Elektrochirurgie-Generator (12) vorhandenen Anzeige (96) für Text erzeugt werden.

8. Elektrochirurgiesystem (10) mit einem elektrochirurgischen Instrument (14), das mindestens einen Instrumenten-Datenspeicher (26) aufweist, und einem Elektrochirurgie-Generator (12), an den das elektrochirurgische Instrument (14) angeschlossen ist, wobei der Elektrochirurgie-Generator (12) über einen Prozessor (70) und mindestens einen Generator-Datenspeicher (72) verfügt, wobei der Prozessor (70) zur Steuerung des Elektrochirurgie-Generators ein in dem Generator-Datenspeicher (72) hinterlegtes Betriebsprogramm (74) ausführt,
wobei in dem Instrumenten-Datenspeicher (26) des elektrochirurgischen Instruments (14) ein Datensatz hinterlegt ist, wobei der in dem Instrumenten-Datenspeicher (26) hinterlegte Datensatz strukturierten Daten enthält, die als eine Vielzahl von parametrisierten Verweisen ausgeführt sind, die auf im Generator-Datenspeicher (72) des Elektrochirurgie-Generators (12) hinterlegte, Steuerbefehle enthaltende Betriebsvorgaben verweisen und/oder bei Bedarf zusätzlichen Betriebsparameter umfassen,
und der Elektrochirurgie-Generator (12) ausgebildet ist,
bei Inbetriebnahme oder während des Betriebs des Elektrochirurgiesystems den im Instrumenten-Datenspeicher (26) des elektrochirurgischen Instruments (14) hinterlegten Datensatz mit strukturierten Daten auszulesen, und
die in den strukturierten Daten hinterlegten parametrisierten Verweise durch das Betriebsprogramm gesteuert aufzurufen, um die im Elektrochirurgie-Generator (12) hinterlegten Steuerbefehle unter Berücksichtigung von in den strukturierten Daten angegebenen Betriebsparametern auszuführen.

9. Elektrochirurgiesystem (10) gemäß Anspruch 8, bei dem der Elektrochirurgie-Generator (12) einen Tongenerator (92) und einen mit diesem zur Ausgabe akustischer Signale verbundenen Lautsprecher (94) aufweist, wobei der Tongenerator (92) mit dem Prozessor (70) wirkverbunden und ausgebildet ist, durch den Prozessor (70) in Abhängigkeit von Signalisierungsanweisungen repräsentierenden Daten in dem Instrumenten-Datenspeicher (26) des elektrochirurgischen Instruments (14) gesteuert akustische Signale zu generieren und über den Lautsprecher (94) auszugeben.

10. Elektrochirurgiesystem (10) gemäß Anspruch 9, bei dem in dem Generator-Datenspeicher (76) Audiodateien gespeichert sind, die der Tongenerator (92) durch den Prozessor (70) und das Betriebsprogramm (74) in Abhängigkeit von Signalisierungsanweisungen repräsentierenden Daten in dem Instrumenten-Datenspeicher (26) des elektrochirurgischen Instruments (14) gesteuert in akustische Signale umsetzen kann.

11. Elektrochirurgiesystem (10) gemäß wenigstens einem der Ansprüche 8 bis 10, bei dem der Elektrochirurgie-Generator (12) eine Anzeige (96) zum Anzeigen von Text und/oder Symbolen aufweist, die mit dem Prozessor (70) verbunden und ausgebildet ist, durch den Prozessor (70) und das Betriebsprogramm (74) in Abhängigkeit von Signalisierungsanweisungen repräsentierenden Daten in dem Instrumenten-Datenspeicher (26) des elektrochirurgischen Instruments (14) gesteuert Textnachrichten auszugeben.

12. Elektrochirurgiesystem (10) gemäß wenigstens einem der Ansprüche 8 bis 11, mit einer Programmierschnittstelle (82) oder mehreren Programmierschnittstellen, mittels der die Inhalte des Generator-Datenspeichers (72) - nämlich vorzugsweise die Betriebsvorgaben (76) - und der Datenstruktur (78) programmiert werden können.

13. Elektrochirurgisches Instrument (14) für ein Elektrochirurgiesystem (10) gemäß der Ansprüche 8 bis 12, mit einem Instrumenten-Datenspeicher (26), der Datensätze (80) mit strukturierten Daten enthält, die Daten enthalten, welche Signalisierungsanweisungen für durch einen Elektrochirurgie-Generator (12) im Betrieb auszugebende akustische und/oder optische Signale repräsentieren.

14. Verfahren zum Erstellen eines strukturierten Datensatzes für ein elektrochirurgisches Instrument (14) eines Elektrochirurgiesystems (10) gemäß der Ansprüche 8 bis 13, **dadurch gekennzeichnet, dass** eine Programmierschnittstelle (82) zur Verfügung gestellt wird und dass über die Programmierschnittstelle (82) ein Datensatz gemäß Anspruch 8, mit strukturierten Daten, die als eine Vielzahl von parametrisierten Verweisen ausgeführt sind, erstellt wird und in dem Instrumenten-Datenspeicher (26) des elektrochirurgischen Instruments (14) gemäß Anspruch 8 abgelegt wird.

## Claims

1. A non-therapeutic method of operating an electrosurgical system (10) comprising an electrosurgical instrument (14) having at least one instrument data memory, and an electrosurgical generator (12) to which the electrosurgical instrument (14) is connected during operation, the electrosurgical generator (12) having a processor (70) and at least one generator data memory (72) connected to the processor (70), wherein the processor (70) executes an operating program (74) stored in the generator data memory (72) to control the electrosurgical generator (12)
a data set is or will be stored in the instrument data memory of the electrosurgical instrument, and
during start-up or operation of the electrosurgical generator (12), the data set stored in the instrument data memory (72) of the electrosurgical instrument (14) is read out by the electrosurgical generator
**characterized in that**
the data set includes structured data, wherein the structured data is implemented as a plurality of parameterized references referring to operating specifications (76) stored in the generator data memory (72) of the electrosurgical generator (12), which comprise potentially applicable control commands and may additionally comprise operating parameter values, and
the parameterized references stored in the structured data are called up under control of the operating program (74) in order to execute the control commands stored in the electrosurgery generator (12), so as to control the electrosurgery generator in dependence on the operating program, the operating specifications stored in the generator data memory and the parameterized references to individual ones of the control commands stored in the generator data memory, that are comprised in the structured data.

2. Method according to claim 1, wherein the data set with structured data stored in the instrument data memory (26) of the electrosurgical instrument (14) is stored in the electrosurgical generator's generator data memory (74) after being read out by the electrosurgical generator.

3. Method according to claim 2, wherein structured data stored in the instrument data memory (26) of the electrosurgical instrument (14) is modified upon readout by the electrosurgical generator (12) or upon storage in the generator data memory (72) of the electrosurgical generator (12).

4. Method according to at least one of the preceding claims, wherein the structured data comprises signaling instructions that are converted during operation of the electrosurgical generator into configuration- and/or operation status-dependent acoustic and/or optical notifications to a user.

5. Method according to at least one of the preceding claims, wherein the structured data comprises definitions for activation tones.

6. Method according to at least one of the preceding claims, wherein the structured data comprises conditions and properties for generating and/or reproducing notifications and/or error messages.

7. Method according to at least one of the preceding claims, wherein the structured data comprises control characters for data representing text messages, said control characters defining the rendering of text messages on different displays, and text messages are generated during operation of the electrosurgical generator (12) in dependence of the control characters and a display (96) for text provided on the electrosurgical generator (12).

8. Electrosurgical system (10) comprising an electrosurgical instrument (14) having at least one instrument data memory (26), and an electrosurgical generator (12) to which the electrosurgical instrument (14) is connected, wherein the electrosurgical generator (12) has a processor (70) and at least one generator data memory (72), wherein the processor (70) executes an operating program (74) stored in the generator data memory (72) for controlling the electrosurgical generator,
wherein a data set is stored in the instrument data memory (26) of the electrosurgical instrument (14), wherein the data set stored in the instrument data memory (26) contains structured data implemented as a plurality of parameterized references that reference operating specifications stored in the generator data memory (72) of the electrosurgical generator (12) comprising control commands and/or comprise additional operating parameters, if required,
and the electrosurgical generator (12) is configured,
upon start-up or during operation of the electrosurgical system, reading out the data set with structured data stored in the instrument data memory (26) of the electrosurgical instrument (14), and
to call up the parameterized references stored in the structured data in a manner controlled by the operating program in order to execute the control commands stored in the electrosurgical generator (12) taking into account operating parameters specified in the structured data.

9. Electrosurgical system (10) according to claim 8, wherein the electrosurgical generator (12) includes a tone generator (92) and a speaker (94) connected thereto for outputting acoustic signals, the tone generator (92) being operatively connected to the processor (70) and adapted to generate acoustic signals controlled by the processor (70) in response to data representing signaling instructions in the instrument data memory (26) of the electrosurgical instrument (14) and to output the acoustic signals through the speaker (94).

10. Electrosurgical system (10) according to claim 9, wherein audio files are stored in the generator data memory (72) and can be converted into acoustic signals by the tone generator (92) under the control of the processor (70) and the operating program (74) in response to data representing signaling instructions in the instrument data memory (26) of the electrosurgical instrument (14).

11. Electrosurgical system (10) according to at least one of claims 8 to 10, wherein the electrosurgical generator (12) comprises a display (96) for displaying text and/or symbols, connected to the processor (70) and adapted to output text messages controlled by the processor (70) and the operating program (74) in response to signaling instructions representing data in the instrument data memory (26) of the electrosurgical instrument (14).

12. Electrosurgical system (10) according to at least one of claims 8 to 11, comprising one or more programming interfaces (82) by means of which the contents of the generator data memory (72) - namely preferably the operating specifications (76) - and the data structure (78) can be programmed.

13. Electrosurgical instrument (14) for an electrosurgical system (10) according to claims 8 to 12, comprising an instrument data memory (26) containing structured data sets (80) containing data representing signaling instructions for acoustic and/or optical signals to be output by an electrosurgical generator (12) during operation.

14. Method for creating a structured data set for an electrosurgical instrument (14) of an electrosurgical system (10) according to claims 8 to 13, **characterized in that** a programming interface (82) is provided and that a data set according to claim 8 comprising structured data embodied as a plurality of parameterized references is created via the programming interface (82) and stored in the instrument data memory (26) of the electrosurgical instrument (14) according to claim 8.

## Revendications

1. Procédé non thérapeutique
pour faire fonctionner un système (10) d'électrochirurgie comprenant un instrument (14) électrochirurgical, qui a au moins une mémoire de donnée d'instrument, et comprenant un générateur (12) d'électrochirurgie, auquel l'instrument (14) électrochirurgical est raccordé en fonctionnement, dans lequel le générateur (12) d'électrochirurgie dispose d'un processeur (70) et d'au moins une mémoire (72) de données de générateur reliée au processeur (70), dans lequel le processeur (72) exécute, pour la commande du générateur (12) d'électrochirurgie, un programme (74) de fonctionnement mis dans la mémoire (72) de donnée de générateur
dans lequel on met ou il est mis un ensemble de données dans la mémoire de données d'instrument de l'instrument électrochirurgical, et,
lors de la mise en fonctionnement ou lors du fonctionnement du générateur (12) d'électrochimie, l'ensemble de données mis dans la mémoire(72) de données d'instrument de l'instrument (14) électrochirurgical est lu par le générateur d'électrochirurgie
**caractérisé en ce que**
l'ensemble de données contient des données structurées, qui sont réalisées sous la forme d'une pluralité de renvois paramétrés et qui renvoient à des prescriptions (76) de fonctionnement, qui sont mises dans la mémoire (72) de données de générateur du générateur (12) d'électrochirurgie et qui peuvent comprendre des instructions de commande à utiliser potentiellement et comprendre supplémentairement des valeurs de paramètres de fonctionnement, et
on appelle, de manière commandée par le programme (74) de fonctionnement, les renvois paramétrés mis dans les données structurées, afin d'exécuter les instructions de commande mises dans le générateur (12) d'électrochirurgie, en tenant compte de paramètres de fonctionnement présents, le cas échéant, dans les données structurées, de manière à commander le générateur d'électrochirurgie en fonction du programme de fonctionnement, des prescriptions de fonctionnement mises dans la mémoire de données de générateur, et des renvois paramétrés, contenus dans les données structurées, à diverses des instructions de commande mises dans la mémoire de données de générateur.

2. Procédé suivant la revendication 1, dans lequel on archive l'ensemble de données, mis dans la mémoire (26) de données d'instrument de l'instrument (14) électrochirurgical, ayant des données structurées, après la lecture par le générateur d'électrochirurgie, dans sa mémoire (74) de données de générateur.

3. Procédé suivant la revendication 2, dans lequel on modifie des données structurées mises dans la mémoire (26) de données de l'instrument (14) électrochirurgical, lors de la lecture par le générateur (12) d'électrochirurgie ou après l'archivage dans la mémoire (72) de données de générateur, du générateur (12) d'électrochirurgie.

4. Procédé suivant au moins l'une des revendications précédentes, dans lequel les données structurées comprennent des instructions de signalisation qui, lorsque le générateur d'électrochirurgie fonctionne, sont transformées en informations acoustiques et/ou optiques de configuration et/ou en fonction de statut de fonctionnement à un utilisateur.

5. Procédé suivant au moins l'une des revendications précédentes, dans lequel les données structurées comprennent des définitions de sons d'activation.

6. Procédé suivant au moins l'une des revendications précédentes, dans lequel les données structurées contiennent des conditions et des propriétés pour la production et/ou la restitution d'informations et/ou de messages d'erreur.

7. Procédé suivant au moins l'une des revendications précédentes, dans lequel des données, représentant les données structurées pour des messages de texte, comprennent des signes de commande, qui définissent la restitution de messages de texte, sur des affichages différents et on produit des messages de texte alors que le générateur (12) d'électrochirurgie est en fonctionnement, en fonction des signes de commande et d'un affichage (96) de texte présent sur le générateur (12) d'électrochirurgie.

8. Système (10) d'électrochirurgie, comprenant un instrument (14) électrochirurgical, qui a au moins une mémoire (26) de données d'instrument, et un générateur (12) d'électrochirurgie, auquel l'instrument (14) électrochirurgical est raccordé, dans lequel le générateur (12) d'électrochirurgie dispose d'un processeur (70) et d'au moins une mémoire (72) de données de générateur, dans lequel le processeur (70) exécute, pour la commande du générateur d'électrochirurgie, un programme (74) de fonctionnement mis dans la mémoire (72) de données de générateur,
dans lequel il est mis, dans la mémoire (26) de données d'instrument de l'instrument (14) électrochirurgical, un ensemble de données, dans lequel l'ensemble de données, mis dans la mémoire (26) de données d'instrument, contient des données structurées, qui sont réalisées sous la forme d'une pluralité de renvois paramétrés, qui renvoient à des prescriptions de fonctionnement mises dans la mémoire (72) de données de générateur du générateur (12) d'électrochirurgie, et contenant des instructions de commandes et/ou comprennent, en cas de besoin, des paramètres de fonctionnement supplémentaires,
et le générateur (12) d'électrochirurgie est constitué
pour, lors de la mise en fonctionnement ou pendant le fonctionnement du système d'électrochirurgie, lire l'ensemble de données mis dans la mémoire (26) d'instrument de l'instrument (14) électrochirurgical et ayant des données structurées, et
pour appeler, de manière commandée par le programme de fonctionnement, les renvois paramétrés mis dans les données structurées, afin d'exécuter les instructions de commande mises dans le générateur (12) d'électrochirurgie, en tenant compte de paramètres de fonctionnement indiqués dans les données structurées.

9. Système (10) d'électrochirurgie suivant la revendication 8, dans lequel le générateur (12) d'électrochirurgie a un générateur (92) de son et un haut-parleur (94) relié à celui-ci pour l'émission de signaux acoustiques, dans lequel le générateur (92) de son est en liaison d'action avec le processeur (70) et est constitué pour créer des signaux acoustiques, de manière commandée par le processeur de signalisation (70) en fonction de données représentant des instructions dans la mémoire (26) de données d'instrument de l'instrument (14) électrochirurgical, et pour les émettre par le haut-parleur (94).

10. Système (10) d'électrochirurgie suivant la revendication 9, dans lequel, dans la mémoire (76) de données de générateur, sont mis en mémoire des fichiers audio, que le générateur (92) de son peut transformer par le processeur (70) et par le programme (74) de fonctionnement en signaux acoustiques, de manière commandée en fonction de données, représentant des instructions de signalisation, dans la mémoire (26) de données d'instrument de l'instrument (14) électrochirurgical.

11. Système (10) d'électrochirurgie suivant au moins l'une des revendications 8 à 10, dans lequel le générateur (12) d'électrochirurgie a un affichage (96) pour l'affichage de texte et/ou de symboles, qui est relié au processeur (70) et est constitué pour émettre, par le processeur (70) et par le programme (74) de fonctionnement, des messages de texte de manière commandées en fonction de données, représentant des instructions de signalisation, dans la mémoire (26) de données d'instrument de l'instrument (14) électrochirurgical.

12. Système (10) d'électrochirurgie suivant au moins l'une des revendications 8 à 11, comprenant une interface (82) de programmation ou plusieurs interfaces de programmation, au moyen de laquelle les contenus de la mémoire (72) de données de générateur - à savoir de préférence les prescriptions (76) de fonctionnement - et la structure (78) de données, peuvent programmées.

13. Instrument (14) électrochirurgical pour un système (10) d'électrochirurgie suivant les revendications 8 à 12, comprenant une mémoire (26) de données d'instrument, qui contient des ensembles (80) de données ayant des données structurées, qui contiennent des données, lesquelles représentent des instructions de signalisation pour des signaux acoustiques et/ou optiques à émettre en fonctionnement par le générateur (12) d'électrochirurgie.

14. Procédé d'établissement d'un ensemble de données structurées, pour un instrument (14) électrochirurgical d'un système (10) d'électrochirurgie suivant les revendications 8 à 13, **caractérisé en ce qu'**on met à disposition une interface (82) de programmation, et **en ce que**, par l'interface (82) de programmation, on établit un ensemble de données suivant la revendication 8, ayant des données structurées, qui sont réalisées sous la forme d'une pluralité de renvois paramétrés, et on l'archive, suivant la revendication 8, dans la mémoire (26) de données d'instrument de l'instrument (14) électrochirurgical.
